# EUROPEAN PATENT APPLICATION

(11) **EP 4 613 288 A1**
(43) Date of publication of application: **10.09.2025**
(21) Application number: 23917011.1
(22) Date of filing: 21.07.2023
(51) Int. Cl.: A61K 39/395, A61K 47/26, A61K 9/08, A61K 47/18, A61K 47/12, A61K 47/02, A61K 47/10, C07K 16/28, A61K 9/00, A61P 7/04

(54) **PREPARATION OF ANTI-APC MONOCLONAL ANTIBODY AND USE THEREOF**

(30) Priority: 20.01.2023 CN 202310062944
(71) Applicant: Oklahoma Medical Research Foundation, Oklahoma City, OK 73104 (US)
(72) Inventor: XU, Jun, Shanghai 201401 (CN); CHENG, Lu, Shanghai 201401 (CN)
(74) Representative: Canzler & Bergmeier Patentanwälte Partnerschaft mbB
(86) International application number: PCT/CN2023/108571
(87) International publication number: WO 2024/152539

(57) **Abstract**

Provided in the present invention is a preparation of an anti-aPC monoclonal antibody. A buffer system of the preparation is histidine/histidine hydrochloride or acetic acid/sodium acetate. The preparation at least comprises two of the following protective agent components: a. a saccharide or a sugar alcohol, the saccharide being selected from trehalose and sucrose, and the sugar alcohol being selected from sorbitol or mannitol; b. an amino acid, the amino acid being selected from arginine hydrochloride or aspartic acid; and c. sodium chloride. The pH of the preparation is 5.0-6.3. Said preparation in the present invention ensures long-term stability of an anti-aPC monoclonal antibody under a preservation condition, thereby obtaining a preparation prescription capable of stabilizing said monoclonal antibody.

## Description

### FIELD OF TECHNOLOGY

The present disclosure relates to the field of biomedicine, specifically to a formulation of an anti-activated protein C (anti-aPC) monoclonal antibody and use thereof.

### BACKGROUND

The anti-activated protein C (anti-aPC) monoclonal antibody is a humanized antibody obtained by humanizing HAPC 1573 and performing screening after mutating single sites or combined sites. The anti-aPC monoclonal antibody specifically binds to and inhibits activated protein C without binding to or inhibiting non-activated protein C. The antibody specifically binds to human-activated protein C without interacting with activated protein C or non-activated protein C from other species. The affinity of the anti-aPC monoclonal antibody is significantly enhanced, and some variants of the anti-aPC monoclonal antibody exhibit a 100-fold increase in affinity compared to HAPC 1573. In Protac-induced APTT coagulation assays, antibody with low concentration (e.g., 1 µg/ml) can achieve superior inhibition of aPC anticoagulant activity, demonstrating the antibody's potential to effectively block the anticoagulant function of the aPC.

However, the stability of anti-aPC monoclonal antibodies remains suboptimal in practical applications. Enhancing the stability of anti-aPC monoclonal antibody formulations during production and application is still a critical challenge.

### SUMMARY

The present disclosure provides a formulation of an anti-activated protein C (aPC) monoclonal antibody, which can enhance the stability of the anti-aPC monoclonal antibody formulation. A buffer system of the formulation is histidine/histidine hydrochloride or acetic acid /sodium acetate. The protective component of the formulation includes at least two of the following:
a. a sugar or sugar alcohol, wherein the sugar is trehalose or sucrose, and the sugar alcohol is sorbitol or mannitol;
b. an amino acid, wherein the amino acid is arginine hydrochloride or aspartic acid;
c. sodium chloride.

The pH of the formulation ranges from 5.0 to 6.3.

In some embodiments, the buffer system is 10 mM histidine/histidine hydrochloride, 20 mM histidine/histidine hydrochloride, or 20 mM acetic acid/sodium acetate. In some embodiments, the buffer system is 10 mM histidine/histidine hydrochloride or 20 mM histidine/histidine hydrochloride.

In some embodiments, the buffer system is 10 mM histidine/histidine hydrochloride. In some embodiments, the content of trehalose ranges from 5 to 15% wt. In some embodiments, the content of sucrose is 8% wt. In some embodiments, the content of sorbitol is 6% wt. In some embodiments, the content of mannitol is 6% wt. In some embodiments, the content of arginine hydrochloride ranges from 50 to 150 mmol/L. In some embodiments, the content of aspartic acid is 10 mmol/L. In some embodiments, the content of sodium chloride ranges from 50 to 150 mmol/L.

In some embodiments, the protective component in the formulation is selected from the group consisting of: 8% trehalose + 100 mmol/L arginine hydrochloride, 6% sorbitol + 100 mmol/L arginine hydrochloride, 6% mannitol + 100 mmol/L arginine hydrochloride, 100 mmol/L arginine hydrochloride + 50 mmol/L sodium chloride, 100 mmol/L arginine hydrochloride + 10 mmol/L aspartic acid, 8% trehalose + 100 mmol/L sodium chloride, 8% trehalose + 100 mmol/L arginine hydrochloride, 8% trehalose + 100 mmol/L arginine hydrochloride + 50 mmol/L sodium chloride, 8% trehalose + 100 mmol/L arginine hydrochloride + 100 mmol/L sodium chloride, and 8% trehalose + 100 mmol/L arginine hydrochloride + 150 mmol/L sodium chloride.

In some embodiments, the protective component in the formulation is 8% trehalose + 100 mmol/L arginine hydrochloride or 100 mmol/L arginine hydrochloride + 50 mmol/L sodium chloride.

In some embodiments, the formulation further includes a surfactant. The surfactant is polysorbate 80 and/or P188.

In some embodiments, the surfactant is 200~500 ppm polysorbate 80, 200~500 ppm P188, or 200 ppm polysorbate 80 + 200 ppm P188.

In some embodiments, the surfactant is selected from 200 ppm polysorbate 80, 300 ppm polysorbate 80, 500 ppm polysorbate 80, 300 ppm P188, 500 ppm P188, and 200 ppm polysorbate 80 + 200 ppm P188.

In some embodiments, the surfactant is 200 ppm polysorbate 80 + 200 ppm P188.

In some embodiments, the anti-aPC monoclonal antibody includes a light chain having an amino acid sequence of SEQ ID NO: 1 and a heavy chain having an amino acid sequence of SEQ ID NO: 2; or the anti-aPC monoclonal antibody includes a light chain having an amino acid sequence of SEQ ID NO: 3 and a heavy chain having an amino acid sequence of SEQ ID NO: 4.

In some embodiments, the anti-aPC monoclonal antibody includes a light chain having an amino acid sequence of SEQ ID NO: 1 and a heavy chain having an amino acid sequence of SEQ ID NO: 2.

In some embodiments, the anti-aPC monoclonal antibody is of the IgG2 or IgG4. In some embodiments, the anti-aPC monoclonal antibody is of the IgG4.

In some embodiments, the concentration of the anti-aPC monoclonal antibody in the formulation ranges from 30 to 70 mg/mL. In some embodiments, the concentration of the anti-aPC monoclonal antibody is 27~33 mg/mL, 45~55 mg/mL, or 63~77 mg/mL.

In some embodiments, the formulation includes 20 mM histidine/histidine hydrochloride, 8% trehalose, 100 mM arginine hydrochloride, 200 ppm polysorbate 80, and the concentration of the anti-aPC monoclonal antibody is 30 mg/mL, with a pH of 5.3. In some embodiments, the formulation includes 20 mM histidine/histidine hydrochloride, 6% sorbitol, 100 mM arginine hydrochloride, 200 ppm polysorbate 80, and the concentration of the anti-aPC monoclonal antibody is 30 mg/mL, with a pH of 5.3. In some embodiments, the formulation includes 20 mM histidine/histidine hydrochloride, 6% mannitol, 100 mM arginine hydrochloride, 200 ppm polysorbate 80, and the concentration of the anti-aPC monoclonal antibody is 30 mg/mL, with a pH of 5.3. In some embodiments, the formulation includes 20 mM histidine/histidine hydrochloride, 100 mM arginine hydrochloride, 50 mM sodium chloride, 200 ppm polysorbate 80, and the concentration of the anti-aPC monoclonal antibody is 30 mg/mL, with a pH of 5.3. In some embodiments, the formulation includes 20 mM histidine/histidine hydrochloride, 100 mM arginine hydrochloride, 10 mM aspartic acid, 200 ppm polysorbate 80, and the concentration of the anti-aPC monoclonal antibody is 30 mg/mL, with a pH of 5.3. In some embodiments, the formulation includes 20 mM histidine/histidine hydrochloride, 8% trehalose, 100 mM sodium chloride, 200 ppm polysorbate 80, and the concentration of the anti-aPC monoclonal antibody is 30 mg/mL, with a pH of 5.3. In some embodiments, the formulation includes 20 mM histidine/histidine hydrochloride, 8% trehalose, 100 mM arginine hydrochloride, 200 ppm polysorbate 80, and the concentration of the anti-aPC monoclonal antibody is 30 mg/mL, with a pH of 5.6.

In some embodiments, the formulation includes a 10 mM histidine/histidine hydrochloride buffer system, 10% trehalose, 100 mM arginine hydrochloride, 200 ppm polysorbate 80, and the concentration of the anti-aPC monoclonal antibody is 30 mg/mL, with a pH of 5.7~6.3.

In some embodiments, the present disclosure provides a use of the anti-aPC monoclonal antibody formulation in one or more of the following:
i) the preparation of a medicament for preventing and/or treating coagulation deficiency or defect disorders;
ii) the preparation of a promoter for enhancing the ability of aPC to cleave histones H3 and H4;
iii) the preparation of a medicament for inhibiting the anticoagulant activity of activated protein C in an individual;
iv) the preparation of a medicament for treating individuals requiring blood coagulation;
vi) the preparation of a medicament for regulating hemostasis in an individual;
vii) the immunological assay analysis for disease or non-disease diagnostic or therapeutic purposes, or the preparation of detection reagents or kits for aPC;
viii) combination with other reagents or drugs.

In some embodiments, the coagulation deficiency or defect disorders include those caused by coagulation factor deficiencies that lead to bleeding and coagulation abnormalities, as well as coagulopathies caused by trauma or surgery.

In some embodiments, the coagulation deficiency or defect disorders include hemophilia, sepsis, and traumatic hemorrhage.

The formulation of the anti-aPC monoclonal antibody of the present disclosure has the following beneficial effects.

The formulation of the present disclosure can strongly inhibit the anticoagulant activity of aPC. According to the protein C activator (protac)-induced APTT assay, the minimal dose of the anti-aPC monoclonal antibody, which can completely inhibit the anticoagulant activity of aPC in human plasma deficient in various coagulation factors, is only 1 µg/mL at most.

Based on the above, after a series of screening and optimization of the buffer system, pH, protective component, and surfactants in the formulation using high-throughput methods, it is shown that the formulation containing 8% trehalose and 100 mM arginine hydrochloride as the protective component demonstrates better stability compared to other formulations, additionally, the formulation containing 200 ppm polysorbate 80 as the surfactant demonstrates better stability compared to other formulations. A comprehensive analysis of physicochemical and appearance reveals that the optimal formulation includes a 10 mM histidine/histidine hydrochloride buffer system, 10% trehalose, 100 mM arginine hydrochloride, 200 ppm polysorbate 80, and a protein concentration of 30 mg/mL, with a pH of 6.0 ± 0.3. This formulation ensures excellent physicochemical and appearance performance and maintains its appearance without the presence of visible particulates under freeze-thaw conditions.

### BRIEF DESCRIPTION OF THE DRAWINGS

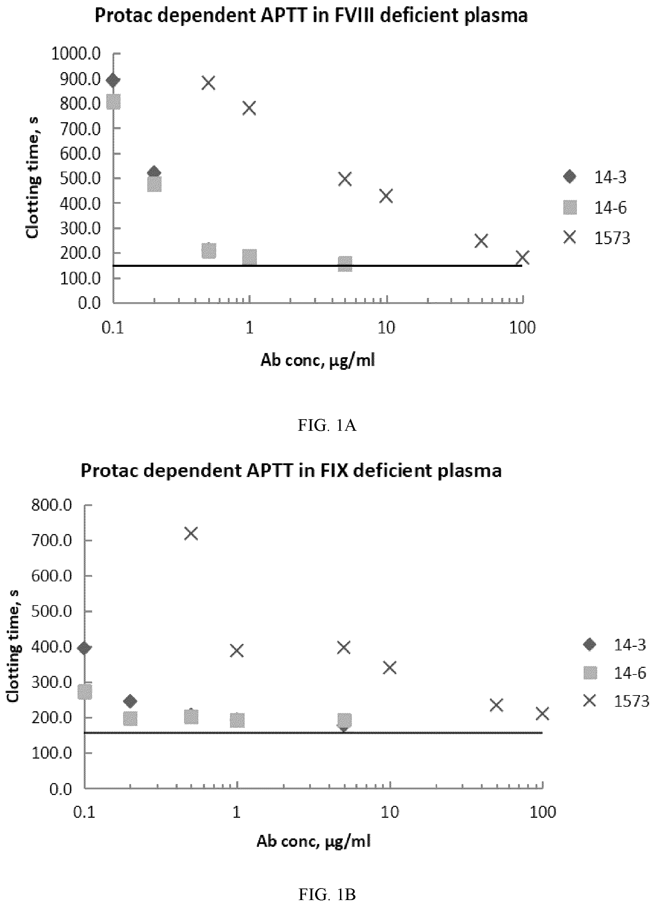

FIG. 1A-1H shows the results of in vitro pharmacodynamic experiments of the present disclosure. The function of the mutant antibody HAPC-14-6 in inhibiting the anticoagulant activity of aPC in coagulation factor-deficient plasma is demonstrated: (A) for FVIII-deficient plasma, (B) for FIX-deficient plasma, (C) for FXI-deficient plasma, (D) for FVII-deficient plasma, (E) for FXII-deficient plasma, (F) for FV-deficient plasma, (G) for FX-deficient plasma, and (H) for VWF-deficient plasma. In FIGS. 1A-1H, HAPC-14-3 is a monoclonal antibody with a variable region sequence carrying mutations different from those of HAPC-14-6, as disclosed in patent application 202110791310X.
FIGS. 2A-2C shows the results of in vivo pharmacodynamic experiments of the present disclosure. FIG. 2A shows the effect of FVIII, HAPC1573, and HAPC-14-6 on the coagulation function of FVIII-deficient mice. FIG. 2B shows the effect of HAPC-14-6 at different doses on the coagulation function of FVIII-deficient mice. FIG. 2C shows the effect of HAPC1573 and HAPC-14-6 at different doses on the coagulation function of FIX-deficient mice.
FIG. 3 shows an exemplary NR-CE detection result for IgG2 subtype-SR602 clones listed in Table 2 of the present disclosure.
FIG. 4 shows the trend of SEC-HPLC monomer changes for each formulation in Table 3 of the present disclosure under 40°C.
FIG. 5 shows the trend of SEC-HPLC aggregate changes for each formulation in Table 3 of the present disclosure under 40°C.
FIG. 6 shows the trend of iCIEF acidic peak changes for each formulation in Table 3 of the present disclosure under 40°C.
FIG. 7 shows the trend of iCIEF main peak changes for each formulation in Table 3 of the present disclosure under 40°C.
FIG. 8 shows the trend of nrCE-SDS main peak changes for each formulation in Table 3 of the present disclosure under 40°C.
FIG. 9 shows the trend of rCE-SDS main peak changes for each formulation in Table 3 of the present disclosure under 40°C.
FIG. 10 shows the trend of SEC-HPLC monomer changes for each formulation in Table 15 of the present disclosure under 40°C.
FIG. 11 shows the trend of SEC-HPLC aggregate changes for each formulation in Table 15 of the present disclosure under 40°C.
FIG. 12 shows the trend of iCIEF acidic peak changes for each formulation in Table 15 of the present disclosure under 40°C.
FIG. 13 shows the trend of iCIEF main peak changes for each formulation in Table 15 of the present disclosure under 40°C.

### DETAILED DESCRIPTION

The present disclosure is further illustrated below with reference to specific embodiments. It should be understood that these embodiments described herein are for illustrative purposes only and shall not be construed as limiting the scope of protection of the present disclosure.

The following describes the implementation of the present disclosure through specific embodiments. Those skilled in the art can readily appreciate other advantages and effects of the present disclosure based on the content disclosed in this specification. The present disclosure may also be implemented or applied in different specific embodiments, and various modifications or changes may be made to the details described herein from different perspectives and applications.

It should be noted that any process equipment or devices not specifically indicated in the following embodiments are conventional equipment or devices in the field. All pressure values and ranges refer to relative pressure.

Furthermore, it should be understood that one or more steps in the present disclosure do not exclude the presence of additional steps before or after the explicitly mentioned steps, or the insertion of other steps between explicitly mentioned steps, unless otherwise specified. Similarly, the combination or connection relationship of one or more devices/apparatuses mentioned in the present disclosure does not exclude the presence of additional devices/apparatuses before or after the explicitly mentioned devices/apparatuses, or the insertion of other devices/apparatuses between the explicitly mentioned devices/apparatuses, unless otherwise specified. Moreover, unless otherwise stated, the numbering of steps is merely a convenient tool for identifying each step and does not limit the sequence of steps or the scope of the invention. Any changes or adjustments to their relative relationships, provided that they do not substantially alter the technical content, shall also be considered within the scope of the present disclosure.

### Example 1 In Vitro Pharmacodynamic Study of Anti-aPC Monoclonal Antibody HAPC-14-6

1.1 The anti-aPC monoclonal antibody used in this example is HAPC-14-6, which is disclosed in Patent Application NO: 202110791310X as the anti-aPC monoclonal antibody HAPC-14-6.

In this example, the anti-aPC monoclonal antibody HAPC-14-6 shares the same light chain variable region and heavy chain variable region as the anti-aPC monoclonal antibodies SR602 and SR604 of the present disclosure, wherein the light chain variable region has an amino acid sequence set forth in SEQ ID NO: 5, and the heavy chain variable region has an amino acid sequence set forth in SEQ ID NO: 6.

HAPC-14-6 is obtained by humanizing HAPC1573 followed by mutation. HAPC-14-6 can bind to haPC but not to hPC, and its affinity is increased by nearly 100-fold compared to the humanized antibody without mutations in the CDR regions. The light chain of HAPC-14-6 is of the kappa subtype.

The preparation of the anti-aPC monoclonal antibody HAPC-14-6 is as follows. Seed cells expressing the monoclonal antibody HAPC-14-6 were constructed using conventional methods (e.g., cloning the genes encoding the antibody heavy and light chains into an expression vector such as pKS001, followed by transfection of the constructed expression vector into CHO cells to obtain seed cells). The anti-aPC monoclonal antibody HAPC-14-6 was obtained after fermentation and purification.

This example evaluates the effects of different concentrations of HAPC1573 and the anti-aPC monoclonal antibody HAPC-14-6 on coagulation factor-deficient plasma.

Using a protac-induced APTT assay, the minimal dose of the monoclonal antibody that can inhibit aPC anticoagulant activity in human plasma deficient in various coagulation factors was determined. The results showed that a concentration of no more than 1 µg/mL was sufficient to inhibit over 95% of aPC anticoagulant activity, whereas HAPC1573 required a concentration exceeding 100 µg/mL.

### 1.2 Experimental Methods and Principles

The principle of Activated Partial Thromboplastin Time (APTT) Assay is as follows. Under 37°C, factors XII and XI were activated by white kaolin, and cephalin (partial thromboplastin) was used as a substitute for platelet factor 3. In the presence of Ca²⁺, the time required for plasma coagulation was measured, which was called the activated partial thromboplastin time. This assay was a sensitive and commonly used screening test for the intrinsic coagulation system.

The newly developed anti-aPC monoclonal antibody HAPC-14-6 can inhibit aPC anticoagulant activity in human plasma deficient in various coagulation factors at a concentration of no more than 1 µg/mL. The various coagulation factors include coagulation factor V, factor VII, factor VIII, factor IX, factor X, factor XI, factor XII, VWF, etc.

### 1.3 Determination of Effects of Antibody on Coagulation Factor-Deficient Plasma

### (1) Experimental Results of Effects of Different Antibodies at Various Concentrations on FVIII and FIX-Deficient Plasma

Antibodies HAPC14-6 and HAPC1573 were diluted to various concentrations (as shown in Table 1) using OWREN-KOLLER buffer.

Experimental samples were established as follows. 150 µL FVIII-deficient plasma was added to 150 µL of a mixed solution containing antibodies at predetermined final concentrations, 0.05 U/ml Protac activator, and OWREN-KOLLER buffer. The samples were thoroughly mixed and incubated at room temperature for 15 min.

A control sample was established by mixing 150 µL of FVIII-deficient plasma with 150 µL of OWREN-KOLLER buffer.

All experimental and control samples were analyzed using the STAGO Compact Max APTT program.

Experimental samples were established as follows. 150 µL FIX-deficient plasma was added to 150 µL of a mixed solution containing antibodies at predetermined final concentrations, 0.01 U/ml Protac activator, and OWREN-KOLLER buffer. The samples were thoroughly mixed and incubated at room temperature for 15 min.

A control sample was established by mixing 150 µL of FIX-deficient plasma with 150 µL of OWREN-KOLLER buffer.

All experimental and control samples were analyzed using the STAGO Compact Max APTT program.

As shown in Table 1, the anti-aPC monoclonal antibody HAPC14-6 exhibited strong inhibition of aPC anticoagulant activity in both FVIII and FIX-deficient plasma. At the same antibody concentration, the inhibitory effect of anti-aPC monoclonal antibody HAPC14-6 on aPC anticoagulant activity was significantly superior to that of murine antibody 1573.

**Table 1**

| Antibodies | Concentration of the APC Antibodies (µg/ml) | Mean Value of the Clotting Time (s) | |
|---|---|---|---|
| | | FVIII-deficient plasma | FIX-deficient plasma |
| 1573 | 5 | 388.9 | 403.5 |
| | 1 | 629 | 566.8 |
| | 0.5 | 600 | 584.2 |
| HAPC 14-6 | 5 | 161.7 | 179.5 |
| | 1 | 186.9 | 198.5 |
| | 0.5 | 182.3 | 181.3 |

### (2) Experimental Results of Effects of Different Antibodies at Various Concentrations on FVIII-Deficient Plasma

Antibodies HAPC-14-3, HAPC-14-6, and HAPC1573 were diluted to various concentrations (as shown in FIG. 1A) using OWREN-KOLLER buffer.

Experimental samples were established as follows. 150 µL FVIII-deficient plasma was added to 150 µL of a mixed solution containing antibodies at predetermined final concentrations, 0.05 U/ml Protac activator, and OWREN-KOLLER buffer. The samples were thoroughly mixed and incubated at room temperature for 15 min.

A control sample was established by mixing 150 µL of FVIII-deficient plasma with 150 µL of OWREN-KOLLER buffer.

All experimental and control samples were analyzed using the STAGO Compact Max APTT program.

As shown in FIG. 1A, the anti-aPC monoclonal antibody completely inhibited the anticoagulant activity of aPC in FVIII-deficient plasma.

### (3) Experimental Results of Effects of Different Antibodies at Various Concentrations on FIX-Deficient Plasma

Antibodies HAPC-14-3, HAPC-14-6, and HAPC1573 were diluted to various concentrations (as shown in FIG. 1B) using OWREN-KOLLER buffer.

Experimental samples were established as follows. 150 µL FIX-deficient plasma was added to 150 µL of a mixed solution containing antibodies at predetermined final concentrations, 0.01 U/ml Protac activator, and OWREN-KOLLER buffer. The samples were thoroughly mixed and incubated at room temperature for 15 min.

A control sample was established by mixing 150 µL of FIX-deficient plasma with 150 µL of OWREN-KOLLER buffer.

All experimental and control samples were analyzed using the STAGO Compact Max APTT program.

As shown in FIG. 1B, the anti-aPC monoclonal antibody completely inhibited the anticoagulant activity of aPC in FIX-deficient plasma.

### (4) Experimental Results of Effects of Different Antibodies at Various Concentrations on FXI-Deficient Plasma

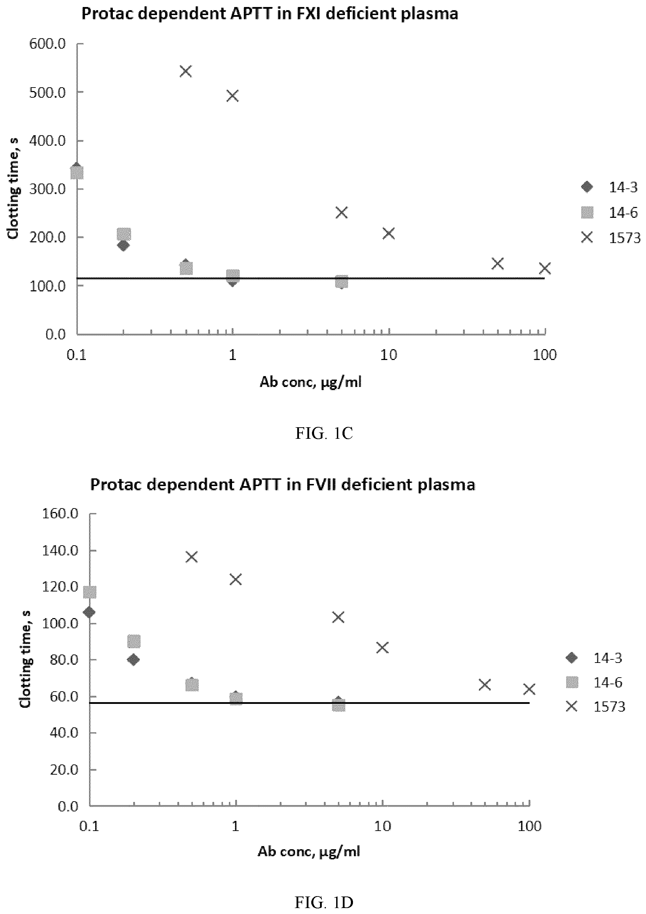

Antibodies HAPC-14-3, HAPC-14-6, and HAPC1573 were diluted to various concentrations (as shown in FIG. 1C) using OWREN-KOLLER buffer.

Experimental samples were established as follows. 150 µL FXI-deficient plasma was added to 150 µL of a mixed solution containing antibodies at predetermined final concentrations, 0.06 U/ml Protac activator, and OWREN-KOLLER buffer. The samples were thoroughly mixed and incubated at room temperature for 15 min.

A control sample was established by mixing 150 µL of FXI-deficient plasma with 150 µL of OWREN-KOLLER buffer.

All experimental and control samples were analyzed using the STAGO Compact Max APTT program.

As shown in FIG. 1C, the anti-aPC monoclonal antibody completely inhibited the anticoagulant activity of aPC in FXI-deficient plasma.

### (5) Experimental Results of Effects of Different Antibodies at Various Concentrations on FVII-Deficient Plasma

Antibodies HAPC-14-3, HAPC-14-6, and HAPC1573 were diluted to various concentrations (as shown in FIG. 1D) using OWREN-KOLLER buffer.

Experimental samples were established as follows. 150 µL FVII-deficient plasma was added with 150 µL of a mixed solution containing antibodies at predetermined final concentrations, 0.1 U/ml Protac activator, and OWREN-KOLLER buffer. The samples were thoroughly mixed and incubated at room temperature for 15 min.

A control sample was established by mixing 150 µL of FVII-deficient plasma with 150 µL of OWREN-KOLLER buffer.

All experimental and control samples were analyzed using the STAGO Compact Max APTT program.

As shown in FIG. 1D, the anti-aPC monoclonal antibody completely inhibited the anticoagulant activity of aPC in FVII-deficient plasma.

### (6) Experimental Results of Effects of Different Antibodies at Various Concentrations on FXII-Deficient Plasma

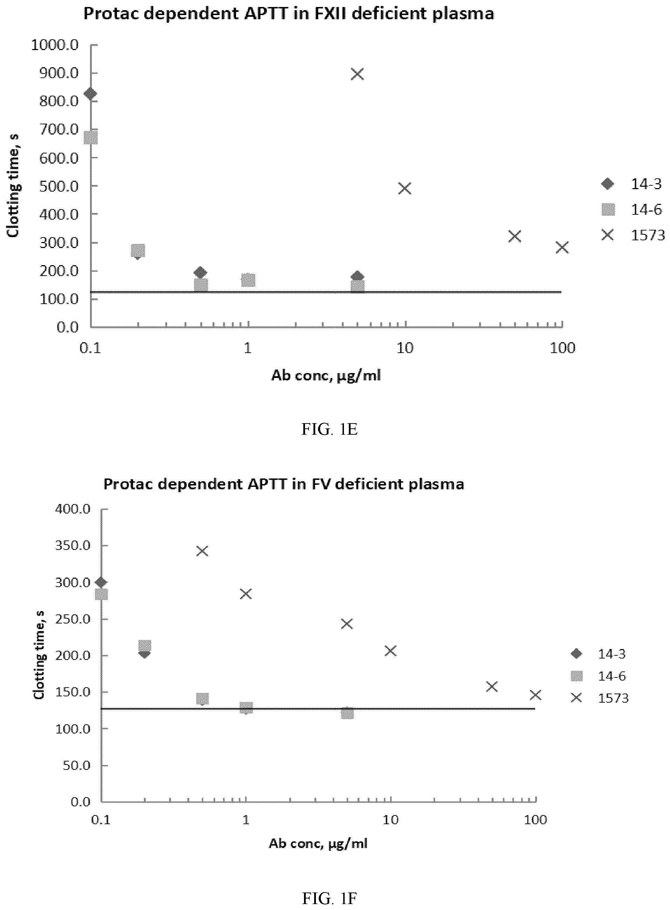

Antibodies HAPC-14-3, HAPC-14-6, and HAPC1573 were diluted to various concentrations (as shown in FIG. 1E) using OWREN-KOLLER buffer.

Experimental samples were established as follows. 150 µL FXII-deficient plasma was added to 150 µL of a mixed solution containing antibodies at predetermined final concentrations, 0.025 U/ml Protac activator, and OWREN-KOLLER buffer. The samples were thoroughly mixed and incubated at room temperature for 15 min.

A control sample was established by mixing 150 µL of FXII-deficient plasma with 150 µL of OWREN-KOLLER buffer.

All experimental and control samples were analyzed using the STAGO Compact Max APTT program.

As shown in FIG. 1E, the anti-aPC monoclonal antibody completely inhibited the anticoagulant activity of aPC in FXII-deficient plasma.

### (7) Experimental Results of Effects of Different Antibodies at Various Concentrations on FV-Deficient Plasma

Antibodies HAPC-14-3, HAPC-14-6, and HAPC1573 were diluted to various concentrations (as shown in FIG. 1F) using OWREN-KOLLER buffer.

Experimental samples were established as follows. 150 µL FV-deficient plasma (containing 5% standard plasma) was added to 150 µL of a mixed solution containing antibodies at predetermined final concentrations, 0.05 U/ml Protac activator, and OWREN-KOLLER buffer. The samples were thoroughly mixed and incubated at room temperature for 15 min.

A control sample was established by mixing 150 µL of FV-deficient plasma (containing 5% standard plasma) with 150 µL of OWREN-KOLLER buffer.

All experimental and control samples were analyzed using the STAGO Compact Max APTT program.

As shown in FIG. 1F, the anti-aPC monoclonal antibody completely inhibited the anticoagulant activity of aPC in FV-deficient plasma.

### (8) Experimental Results of Effects of Different Antibodies at Various Concentrations on FX-Deficient Plasma

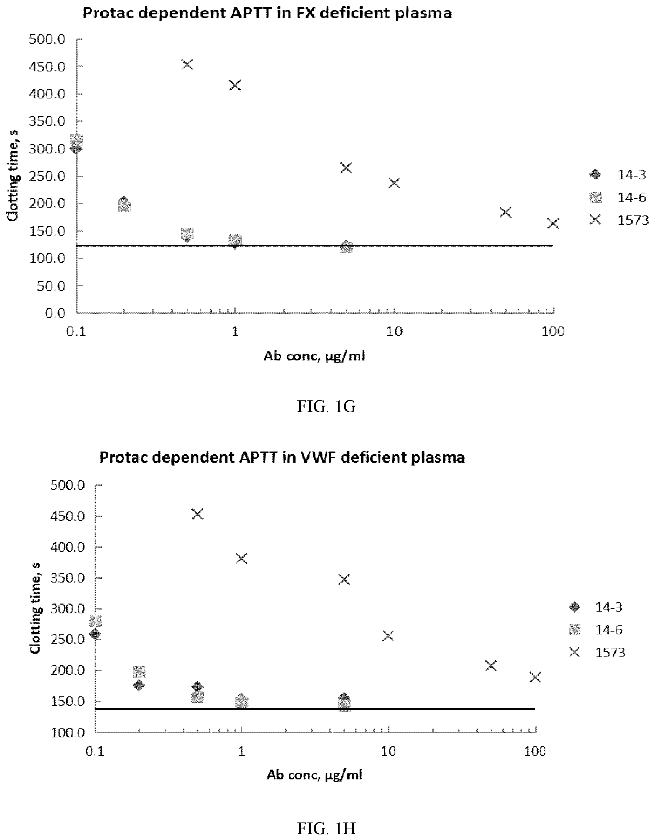

Antibodies HAPC-14-3, HAPC-14-6, and HAPC1573 were diluted to various concentrations (as shown in FIG. 1G) using OWREN-KOLLER buffer.

Experimental samples were established as follows. 150 µL FX-deficient plasma (containing 1% standard plasma) was added to 150 µL of a mixed solution containing antibodies at predetermined final concentrations, 0.05 U/ml Protac activator, and OWREN-KOLLER buffer. The samples were thoroughly mixed and incubated at room temperature for 15 min.

A control sample was established by mixing 150 µL of FX-deficient plasma (containing 1% standard plasma) with 150 µL of OWREN-KOLLER buffer.

All experimental and control samples were analyzed using the STAGO Compact Max APTT program.

As shown in FIG. 1G, the anti-aPC monoclonal antibody completely inhibited the anticoagulant activity of aPC in FX-deficient plasma.

### (9) Experimental Results of Effects of Different Antibodies at Various Concentrations on VWF-Deficient Plasma

Antibodies HAPC-14-3, HAPC-14-6, and HAPC1573 were diluted to various concentrations (as shown in FIG. 1H) using OWREN-KOLLER buffer.

Experimental samples were established as follows. 150 µL VWF-deficient plasma was added to 150 µL of a mixed solution containing antibodies at predetermined final concentrations, 0.01 U/ml Protac activator, and OWREN-KOLLER buffer. The samples were thoroughly mixed and incubated at room temperature for 15 min.

A control sample was established by mixing 150 µL of VWF-deficient plasma with 150 µL of OWREN-KOLLER buffer.

All experimental and control samples were analyzed using the STAGO Compact Max APTT program.

As shown in FIG. 1H, the anti-aPC monoclonal antibody completely inhibited the anticoagulant activity of aPC in VWF-deficient plasma.

### Example 2 In Vivo Pharmacodynamic Study of Anti-aPC Monoclonal Antibody

In a humanized protein C hemophilia mouse model (refer to paragraphs [0230]~[0238] of Patent Application CN201911027061.6), after injection of a specified dose of anti-aPC monoclonal antibody HAPC-14-6, the bleeding volume following tail snip was examined to evaluate the hemostatic effect achieved by the anti-aPC monoclonal antibody through inhibition of aPC anticoagulant function.

### 2.1 Experimental Methods

Administration was performed via retro-orbital venous injection in mice. One hour post administration, the mice were anesthetized by intraperitoneal injection of sodium pentobarbital. Following anesthesia, the tail was snipped 2 mm from the tip and immersed in 15 mL of 37°C physiological saline to collect shed blood for 20 minutes. The collected fluid was subjected to erythrocyte lysis and centrifugation to obtain supernatant. The supernatant was measured at 600 nm wavelength using a UV spectrophotometer. The optical density (OD) value was used to quantify bleeding volume, indicating the coagulation function of the mice. WT denotes wild-type mice, while "untreated" refers to non-dosed humanized protein C hemophilia A or B mice.

### 2.2 Experimental Results

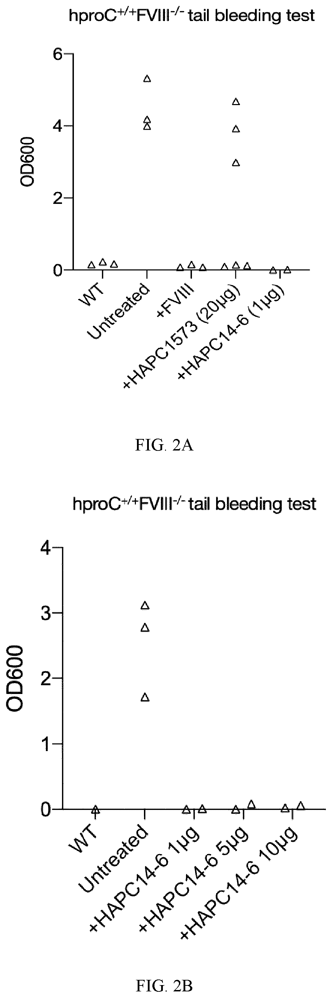

As shown in FIG. 2A, in humanized protein C hemophilia A mice, administration of 1 IU/mouse FVIII, 20 µg/mouse murine antibody 1573, or 1 µg/mouse HAPC-14-6 demonstrated that mice injected with the novel anti-aPC monoclonal antibody HAPC-14-6 restored coagulation function to wild-type levels, exhibiting that the anti-aPC monoclonal antibody HAPC-14-6 had comparable efficacy to FVIII.

FIG. 2B illustrated that in humanized protein C hemophilia A mice, administration of different doses of HAPC-14-6 was performed. The results showed that a maximum of 1 µg HAPC-14-6 could restore the coagulation function of mice to normal levels.

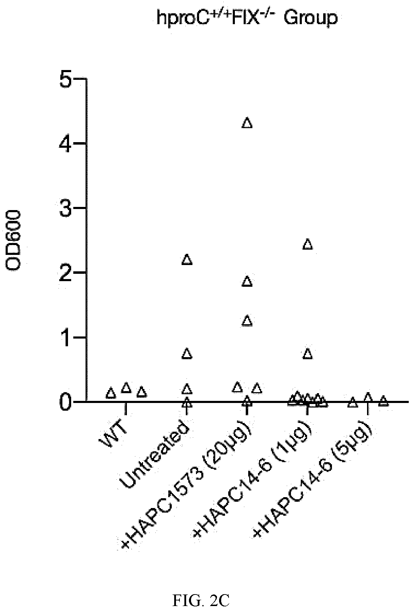

FIG. 2C demonstrated that in humanized protein C hemophilia B mice, administration of different doses of HAPC-14-6 was performed. The results showed that a maximum of 1 µg HAPC-14-6 could restore the coagulation function of mice to normal levels.

The protac-induced APTT assay in the present disclosure, which is for detecting minimal inhibitory concentrations of anti-aPC monoclonal antibodies towards various coagulation factor-deficient human plasmas, revealed that concentrations as low as 1 µg/mL can completely inhibit aPC anticoagulant activity, whereas murine antibody HAPC1573 required concentrations exceeding 100 µg/mL.

### Example 3 Monoclonal Antibody Screening Experiment

Three Minipools with identical HAPC-14-6 variable regions but different IgG subtypes (IgG2 subtype and IgG4 subtype) were selected for monoclonal plating and a series of clone screening experiments. Ultimately, 10 high-expression monoclonal cell lines producing full-length monoclonal antibodies were selected for each subtype. The monoclonal antibody with the IgG4 subtype has a kappa subtype light chain, wherein a light chain of this monoclonal antibody has an amino acid sequence set forth in SEQ ID NO: 1, a heavy chain of this monoclonal antibody has an amino acid sequence set forth in SEQ ID NO: 2, and this monoclonal antibody was designated as SR604. The monoclonal antibody with the IgG2 subtype has a kappa subtype light chain, wherein a light chain of this monoclonal antibody has an amino acid sequence set forth in SEQ ID NO: 3, a heavy chain of this monoclonal antibody has an amino acid sequence set forth in SEQ ID NO: 4, and this monoclonal antibody was designated as SR602.

For the preparation of anti-aPC monoclonal antibodies, seed cells expressing monoclonal antibodies were constructed using conventional methods. Specifically, the genes encoding the full-length heavy chain and light chain of the antibody were cloned into an expression vector such as pKS001, and the constructed expression vector was then transfected into CHO cells to obtain seed cells. The seed cells were inoculated into a 200 L fermenter. After cultivation, the fermentation broth was purified through affinity chromatography, low-pH virus inactivation, intermediate product depth filtration, anion exchange chromatography, cation exchange chromatography, Nanofiltration (NF), ultrafiltration/diafiltration (UF/DF), and drug substance preparation processes to obtain samples meeting the quality standards for drug substances.

The overall recovery rate of this production process was 71.5%. The final purified drug substance (SR604) exhibited the following characteristics: SEC-HPLC analysis showed aggregates at 0.2%, main peak at 99.6%, and fragments at 0.3%; iCIEF analysis revealed acidic peaks at 23.9%, main peak at 64.7%, and basic peaks at 11.3%; HCP residue was 14 ppm; HC-DNA residue was 0 ppb; ProA residue was less than 2 ppm; nrCE-SDS was 97.6%; and rCE-SDS (HC+LC) was 96.2%.

In terms of expression levels, the IgG2 subtype clones exhibited higher expression than the IgG4 subtype, with the highest reaching 7.7 g/L. NR-CE analysis of the expressed products revealed split peaks (i.e., main peak 1 and main peak 2) for the IgG2 subtype expression products. The main peak data for each clone are shown in Table 2:

**Table 2 NR-CE Test Results of 10 Clones of IgG2 Subtype-SR602**

| **Clone** | **Name** | **Peak Area (%)** | **Total Peak Area (%)** | **Clone** | **Name** | **Peak Area (%)** | **Total Peak Area (%)** |
|---|---|---|---|---|---|---|---|
| Number 1 | Main Peak 1 | 53.62 | 97.01 | Number 6 | Main Peak 1 | 55.57 | 96.17 |
| | Main Peak 2 | 43.39 | | | Main Peak 2 | 40.60 | |
| Number 2 | Main Peak 1 | 53.77 | 95.58 | Number 7 | Main Peak 1 | 52.91 | 96.77 |
| | Main Peak 2 | 41.81 | | | Main Peak 2 | 43.86 | |
| Number 3 | Main Peak 1 | 55.83 | 96.49 | Number 8 | Main Peak 1 | 55.00 | 96.33 |
| | Main Peak 2 | 40.66 | | | Main Peak 2 | 41.33 | |
| Number 4 | Main Peak 1 | 53.35 | 96.36 | Number 9 | Main Peak 1 | 53.75 | 96.07 |
| | Main Peak 2 | 43.01 | | | Main Peak 2 | 42.31 | |
| Number 5 | Main Peak 1 | 53.72 | 96.75 | Number 10 | Main Peak 1 | 55.12 | 96.08 |
| | Main Peak 2 | 43.03 | | | Main Peak 2 | 40.95 | |

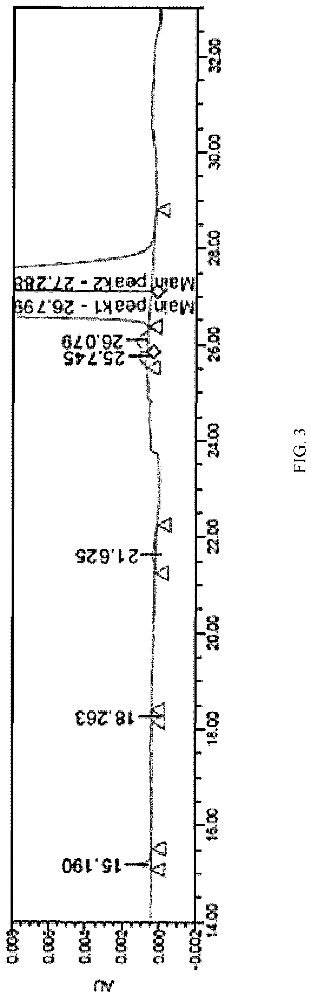

For the 10 monoclonal cell lines corresponding to IgG4 molecular screening, their expression products underwent comprehensive physicochemical characterization tests. The test results were as follows: SEC analysis showed no significant aggregation of the antibodies; CIEF results indicated a PI of 7.2, with no abnormal peaks in the acidic or basic regions; LC-MS analysis indicated that the molecular weight of the antibodies was consistent with the design specifications; HPLC quantification data showed approximately 10% variation compared to Octet Titer detection data, which did not affect clone selection. The highest expression level of IgG4 subtype-clones exceeded 5 g/L, while the remaining clones achieved 3-4 g/L, making them suitable for subsequent production. FIG. 3 shows representative NR-CE test results for the IgG2 subtype-SR602 clones from Table 2 of the present disclosure.

The above comprehensive analysis indicates that the anti-aPC monoclonal antibody SR604 (IgG4 subtype) is superior to the SR602 (IgG2 subtype).

### Example 4 Protective Component Screening Experiment

This example used the anti-aPC monoclonal antibody SR604 verified in Example 3 for protective component screening.

The experiment employed anti-aPC monoclonal antibody at a protein concentration of 30 mg/mL, 20 mM histidine/histidine hydrochloride as the basic buffer system, and pH of 5.3 to screen different protective components.

**Table 3 Different Protective Components**

| **Formulation No.** | **Buffer System** | **pH** | **Protein concentration (g/L)** | **Protective Components** | **Surfactant** |
|---|---|---|---|---|---|
| A | 20 mM Histidine/ Histidine hydrochloride | 5.3 | 30 | 8% Trehalose + 100 mM Arginine hydrochloride | 200 ppm Polysorbate 80 |
| B | | | | 6% Sorbitol + 100 mM Arginine hydrochloride | |
| C | | | | 6% Mannitol + 100 mM Arginine hydrochloride | |
| D | | | | 100 mM Arginine hydrochloride + 50 mM NaCl | |
| E | | | | 100 mM Arginine hydrochloride + 10 mM Aspartic Acid | |
| F | | | | 8% Trehalose + 100 mM NaCl | |
| G | | 5.6 | | 8% Trehalose + 100 mM Arginine hydrochloride | |

The formulations A-G from Table 3 were subjected to the stability study conditions specified in Table 4 for evaluation.

**Table 4 Stability Study Conditions**

| **Study Condition** | **0-point** | **1-week** | **2-week** | **5-week** | **6-week** |
|---|---|---|---|---|---|
| 2~8°C | + | - | - | + | + |
| 25±2°C/60%±5% RH | | - | + | + | + |
| 40±2°C/75%±5% RH | | + | + | + | + |
| Shaking (500 rpm at Room Temperature) | | 3-day | | 7-day | |
| | | + | | + | |
| Freeze-thaw (-80°C freeze for 24h then thaw in 30°C water bath, counted as one cycle) | | 3 cycles | | 6 cycles | |
| | | + | | + | |

The formulations A-G were sampled at the time points shown in Table 4 to evaluate pH (0-point only), viscosity (0-point only), appearance, protein concentration, SEC-HPLC, charge distribution (iCIEF), rCE-SDS purity, and nrCE-SDS purity. The test results are shown in Tables 5~11.

**Table 5 pH and Viscosity Results**

| **Formulation No.** | **pH** | **Viscosity (mPa·s)** |
|---|---|---|
| A | 5.31 | 1.687 |
| B | 5.36 | 1.655 |
| C | 5.35 | 1.670 |
| D | 5.36 | 1.375 |
| E | 5.40 | 1.389 |
| F | 5.40 | 2.016 |
| G | 5.65 | 1.678 |

As shown in Table 5, the viscosity of all seven formulations did not exceed 3 mPa·s, indicating that the addition of arginine hydrochloride or NaCl had a certain effect on reducing protein viscosity.

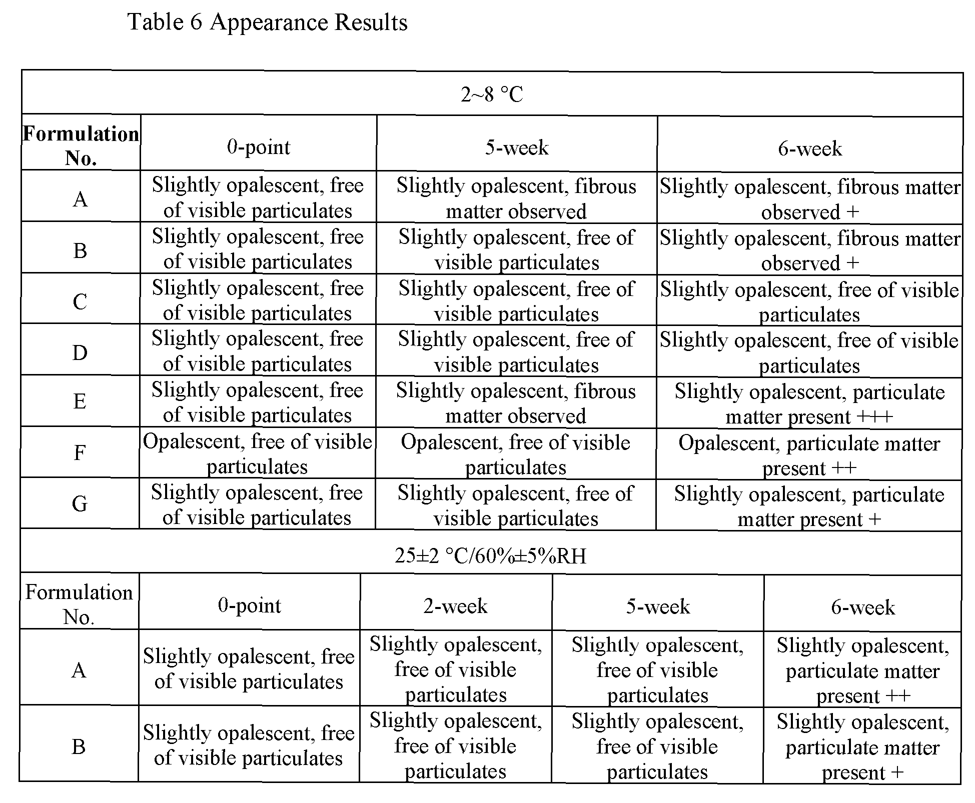

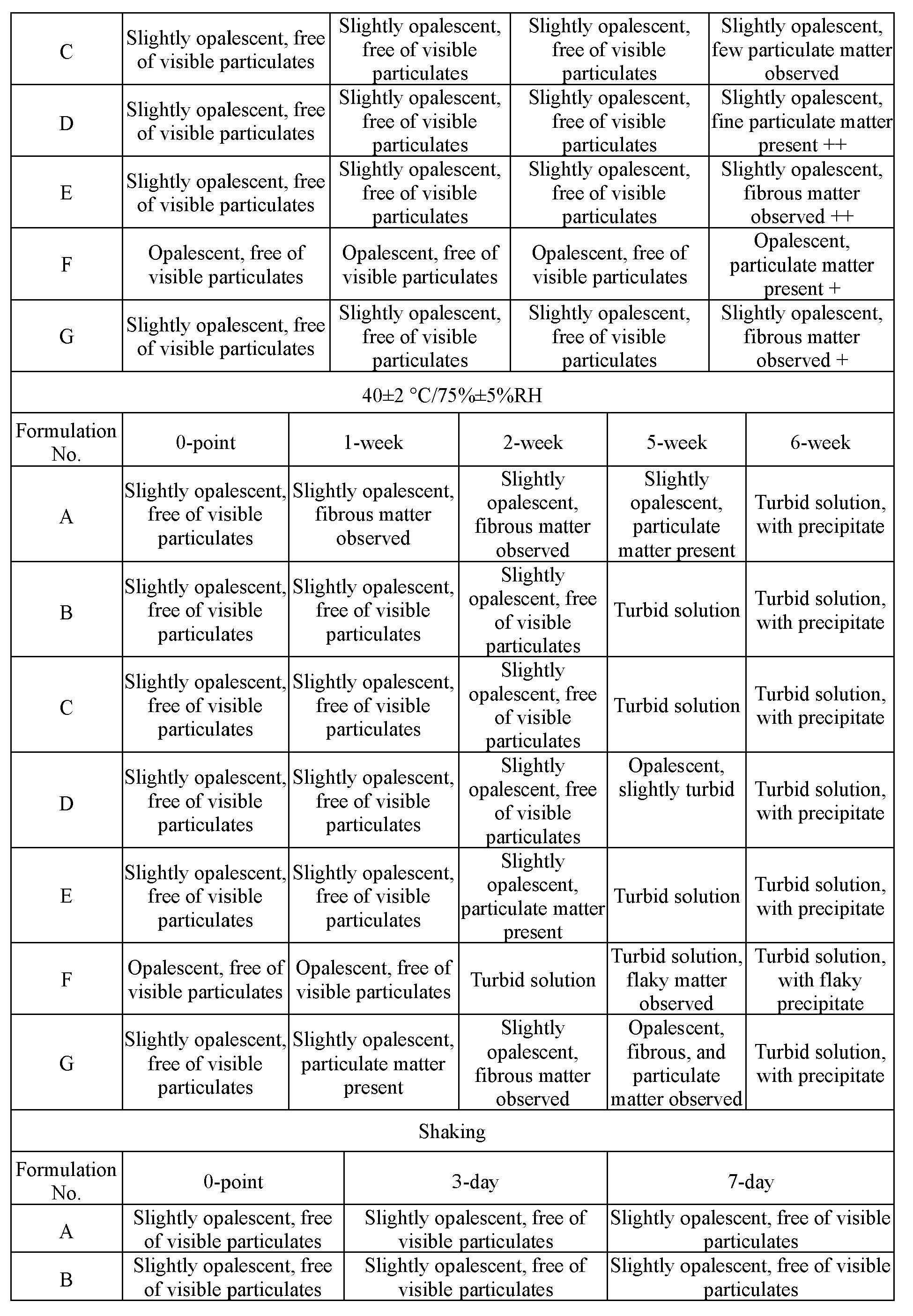

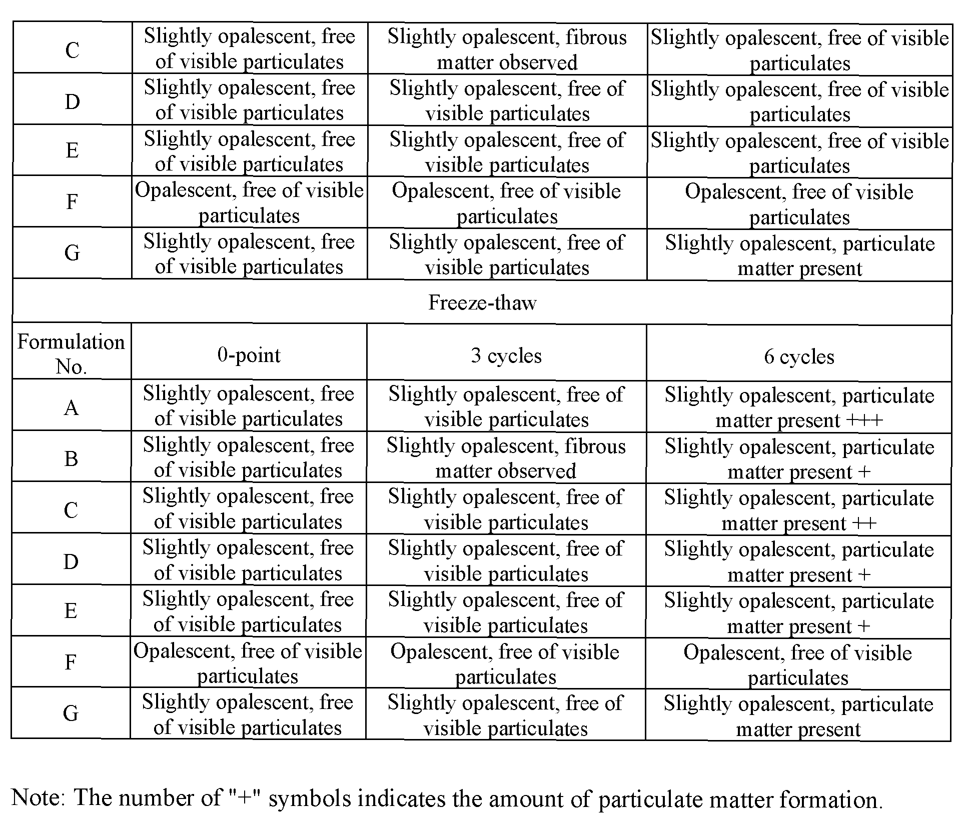

As shown in Table 6, the protein solutions under the seven formulation conditions (A-G) exhibited varying degrees of visible particulates after 6 weeks of storage at 25°C and 40°C. At 2~8°C, only formulations C (6% mannitol + 100 mM arginine hydrochloride) and D (100 mM arginine hydrochloride + 50 mM NaCl) showed no visible particulates after 6 weeks. Formulation A (8% trehalose + 100 mM arginine hydrochloride) exhibited weaker opalescence after storage at 40°C. Except for formulation F (8% trehalose + 100 mM NaCl), all other formulations developed varying levels of visible particulates after 6 cycles of freeze-thaw. These results indicate that formulations A-G can maintain appearance stability for a certain period.

**Table 7 Protein Concentration Results**

| Formul ation No. | 0-point | 2~8°C | | 25±2°C/60%±5%RH | | | 40±2°C/75%±5%RH | | | | Freeze-thaw | | Shaking | |
|---|---|---|---|---|---|---|---|---|---|---|---|---|---|---|
| | | 5-week | 6-week | 2-week | 5-week | 6-week | 1-week | 2-week | 5-week | 6-week | 3 cycles | 6 cycles | 3-day | 7-day |
| A | 28.7 | 27.7 | 27.8 | 28.0 | 27.8 | 28.0 | 28.1 | 27.7 | 27.9 | 28.3 | 28.4 | 28.1 | 28.3 | 28.1 |
| B | 29.1 | 28.3 | 28.3 | 28.2 | 28.3 | 28.3 | 28.6 | 28.5 | 28.4 | 28.6 | 28.6 | 28.1 | 28.5 | 28.4 |
| C | 28.4 | 28.3 | 28.2 | 28.3 | 28.3 | 28.3 | 28.4 | 28.5 | 28.0 | 28.5 | 28.5 | 28.0 | 28.8 | 28.3 |
| D | 29.9 | 29.2 | 28.8 | 29.4 | 29.2 | 29.2 | 29.5 | 29.4 | 28.9 | 29.3 | 29.5 | 29.5 | 29.6 | 29.5 |
| E | 29.3 | 28.7 | 29.3 | 29.1 | 28.9 | 29.1 | 29.3 | 29.2 | 29.0 | 31.7 | 29.2 | 29.1 | 29.6 | 29.3 |
| F | 28.7 | 28.0 | 28.1 | 28.4 | 28.2 | 28.3 | 28.4 | 28.4 | 27.5 | 27.8 | 28.4 | 28.4 | 28.2 | 28.4 |
| G | 28.5 | 28.0 | 27.9 | 28.0 | 28.0 | 28.0 | 28.5 | 28.1 | 28.1 | 28.1 | 28.3 | 28.3 | 28.5 | 28.3 |

As shown in Table 7, the protein concentration of samples under the seven formulation conditions (A-G) showed no significant changes compared to the initial time point (0-point) under all test conditions.

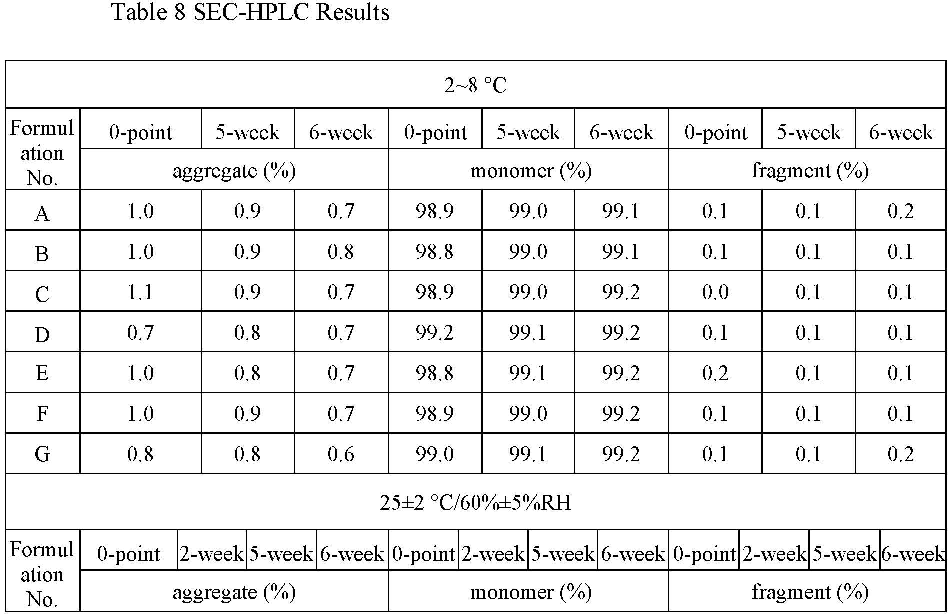

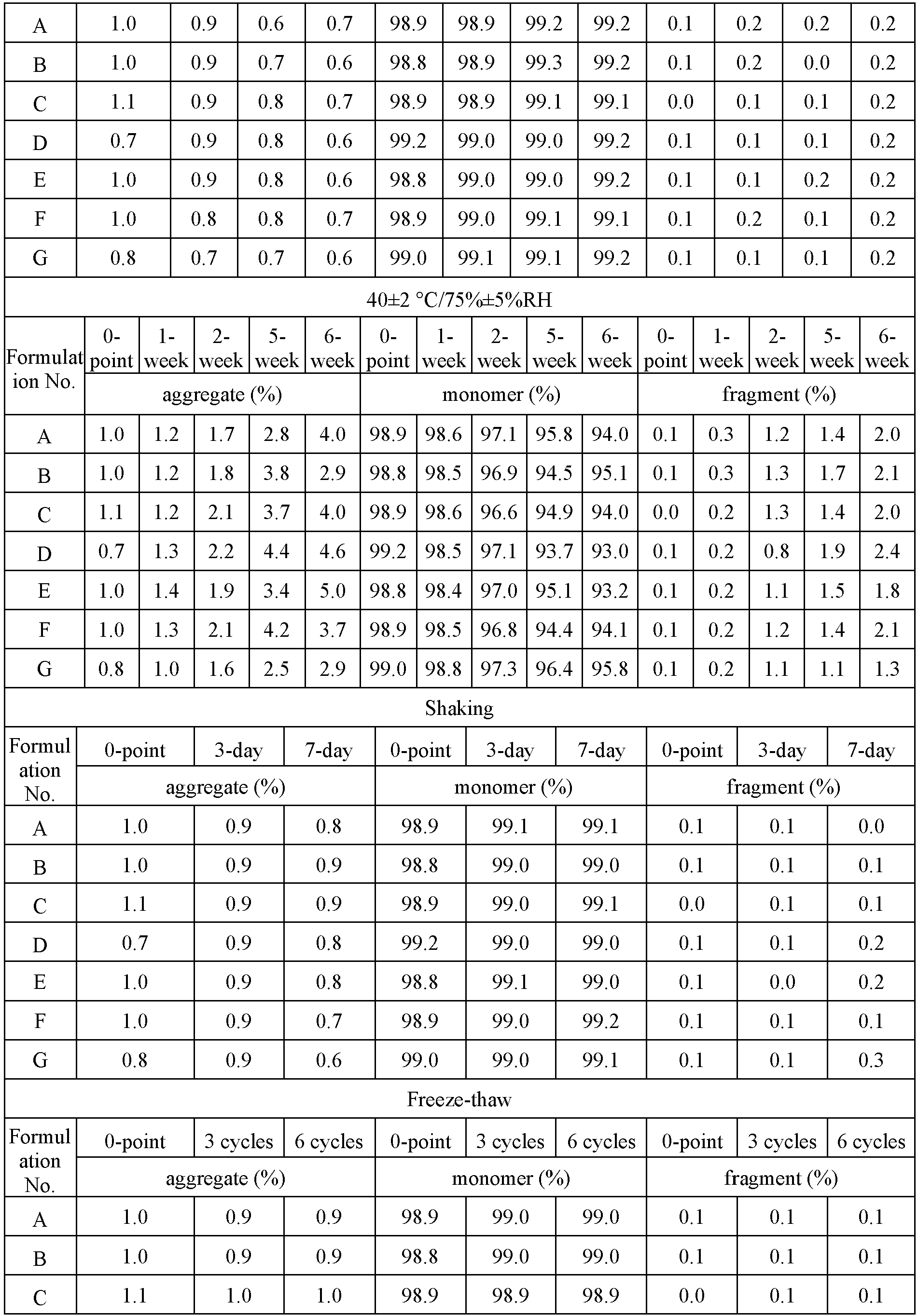

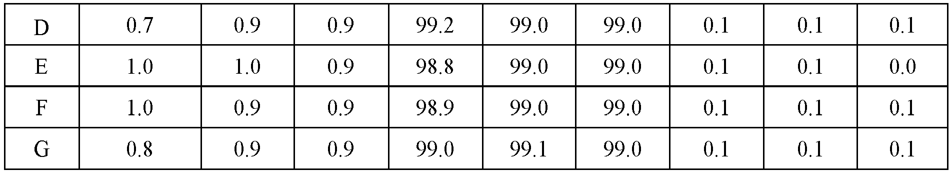

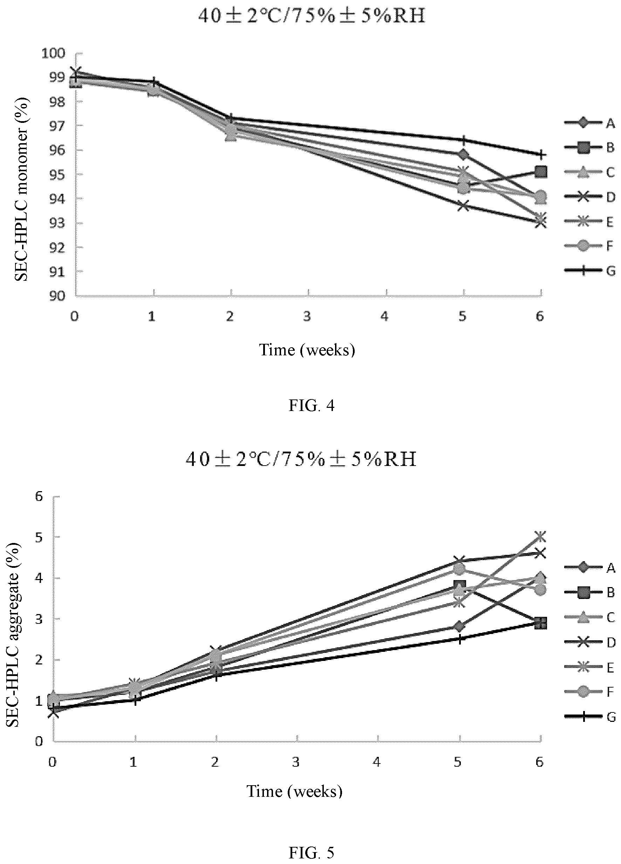

For the seven formulations (A-G), the size exclusion chromatography (SEC) results under conditions of 2~8°C, 25°C, shaking, and freeze-thaw cycles showed no significant changes compared to the 0-point, with no notable differences observed between formulations. FIGS. 4 and 5 demonstrated that under 40°C conditions, all formulations exhibited a significant increase in SEC aggregates and a clear decreasing trend in monomers.

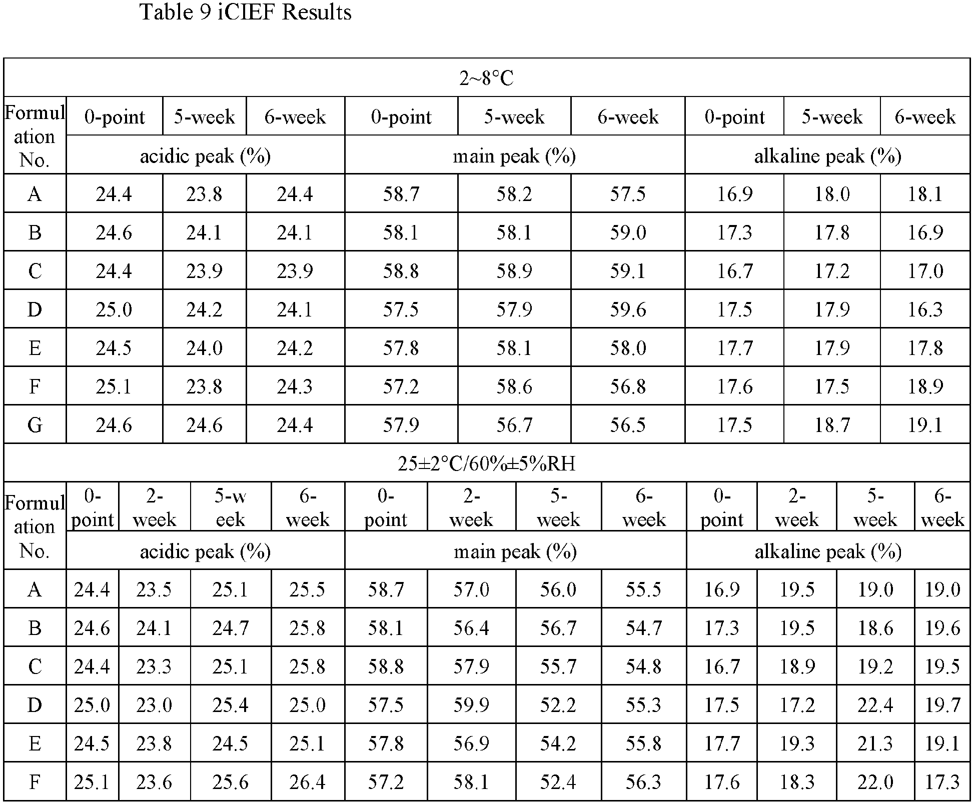

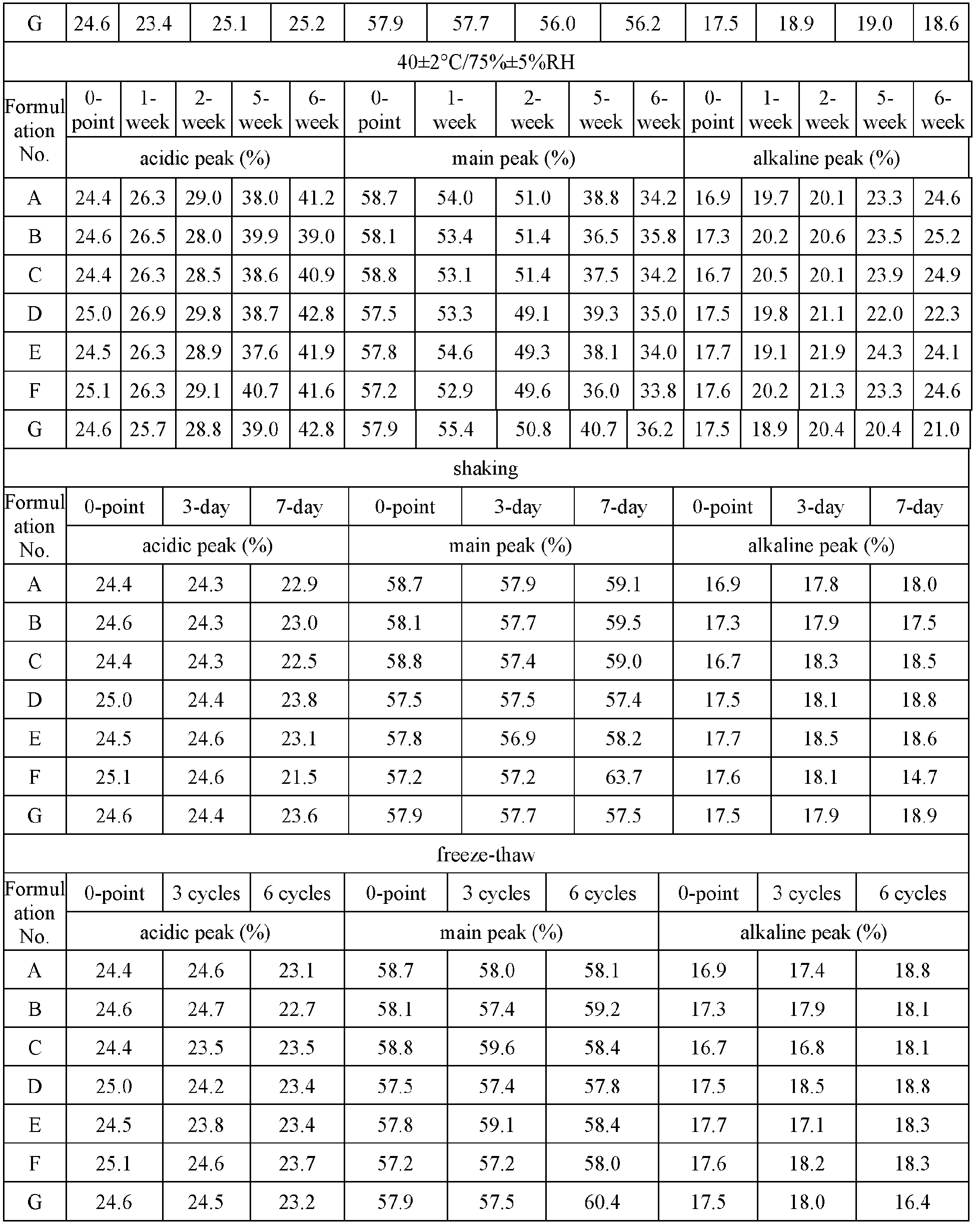

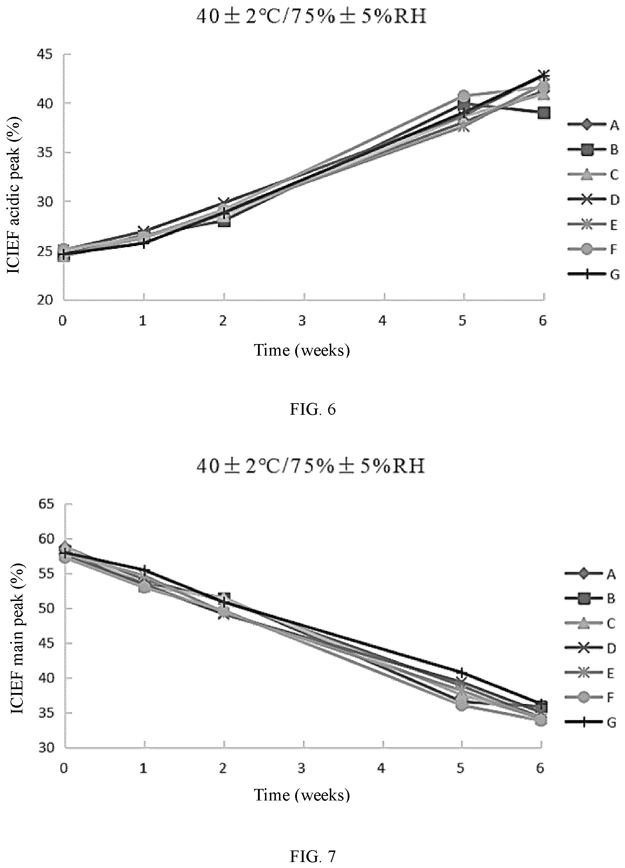

The iCIEF results of the seven formulation samples under 2~8°C, 25°C, shaking, and freeze-thaw conditions showed no significant changes compared to the 0-point, with no notable differences observed between formulations. FIGS. 6 and 7 demonstrated that under 40°C conditions, all formulations exhibited a significant increase in iCIEF acidic peaks and a clear decreasing trend in the main peak, with similar magnitudes of change across formulations.

**Table 10 nrCE-SDS Purity Results**

| Formula tion No. | 0-point | 2~8°C | 25±2°C/60%±5%RH | | 40±2°C/75%±5%RH | | | freeze-thaw | shaking |
|---|---|---|---|---|---|---|---|---|---|
| | | 6-week | 2-week | 6-week | 1-week | 2-week | 6-week | 3 cycles | 3-day |
| A | 97.2 | 96.9 | 97.3 | 96.1 | 95.7 | 94.9 | 83.1 | 97.5 | 97.4 |
| B | 97.2 | 97.1 | 97.3 | 96.4 | 96.7 | 94.7 | 84.5 | 97.4 | 97.2 |
| c | 96.8 | 97.1 | 97.4 | 96.4 | 96.6 | 94.5 | 81.7 | 97.5 | 96.9 |
| D | 96.8 | 97.0 | 97.5 | 96.4 | 96.1 | 94.1 | 81.1 | 96.8 | 97.3 |
| E | 97.6 | 96.8 | 97.4 | 96.2 | 96.3 | 94.4 | 81.2 | 97.6 | 96.9 |
| F | 97.1 | 97.0 | 97.1 | 96.0 | 96.2 | 93.7 | 85.3 | 96.8 | 97.1 |
| G | 96.9 | 97.3 | 97.3 | 95.8 | 96.4 | 96.5 | 86.5 | 97.2 | 97.0 |

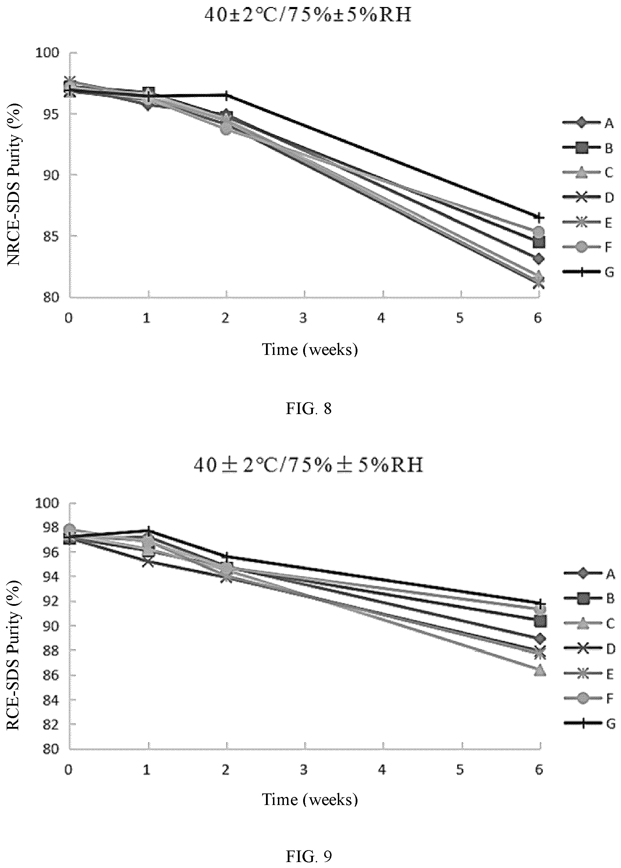

The nrCE-SDS results of the seven formulations under conditions of 2~8°C, 25°C, shaking, and freeze-thaw cycles showed no significant changes compared to the 0-point, with no notable differences observed between formulations. FIG. 8 demonstrated that under 40°C conditions, all formulations exhibited a clear decreasing trend in the nrCE-SDS main peak. Formulations C (6% mannitol + 100 mM arginine hydrochloride), D (100 mM arginine hydrochloride + 50 mM NaCl), and E (100 mM arginine hydrochloride + 10 mM aspartic acid) showed relatively larger declines in the main peak intensity.

**Table 11 rCE-SDS Purity Results**

| Formulation No. | 0-point | 2~8°C | 25±2°C/60%±5%RH | | 40±2°C/75%±5%RH | | | freeze-thaw | shaking |
|---|---|---|---|---|---|---|---|---|---|
| | | 6-week | 2-week | 6-week | 1-week | 2-week | 6-week | 3 cycles | 3-day |
| A | 97.0 | 97.3 | 97.2 | 97.2 | 97.2 | 94.8 | 88.9 | 97.2 | 97.4 |
| B | 97.1 | 97.4 | 97.1 | 97.0 | 96.1 | 94.7 | 90.4 | 97.5 | 97.5 |
| C | 97.4 | 96.8 | 96.8 | 97.0 | 96.2 | 94.5 | 86.4 | 97.4 | 97.6 |
| D | 97.1 | 96.8 | 97.2 | 96.9 | 95.2 | 93.6 | 87.9 | 97.8 | 97.4 |
| E | 97.3 | 97.1 | 96.7 | 96.8 | 96.9 | 94.1 | 87.7 | 97.5 | 97.4 |
| F | 97.8 | 97.1 | 97.1 | 97.1 | 96.8 | 94.8 | 91.3 | 97.5 | 97.6 |
| G | 97.2 | 97.3 | 97.0 | 96.8 | 97.7 | 95.6 | 91.8 | 97.5 | 97.3 |

The rCE-SDS results of the seven formulation samples under 2~8°C, 25°C, shaking, and freeze-thaw conditions showed no significant changes compared to the 0-point, with no notable differences observed between formulations. FIG. 9 indicated that under 40°C conditions, all formulations exhibited a clear decreasing trend in the main peak, with formulations C (6% mannitol + 100 mM arginine hydrochloride), D (100 mM arginine hydrochloride + 50 mM NaCl), and E (100 mM arginine hydrochloride + 10 mM aspartic acid) showing relatively greater decreases.

The comprehensive comparison of physicochemical and appearance results revealed that the addition of various sugars and arginine hydrochloride-based protective components in the formulations can, to some extent, inhibit protein aggregation and precipitation, while reducing the viscosity of the drug solution to a controllable range below 3 mPa·s. Formulation A (8% trehalose + 100 mM arginine hydrochloride) demonstrated optimal stability compared to the other formulations. However, since all formulations developed visible particulates after 6 weeks of storage at 25°C and 40°C, further optimization of the formulations is required to enhance the long-term stability of the protein.

### Example 5 High-Throughput Screening Experiment

### 2.1 Screening Study of Buffer System and pH

This example employed high-throughput rapid screening methods such as UNcle and microplate readers to further optimize formulation A (8% trehalose + 100 mM arginine hydrochloride), in which the protein concentration is increased to 50 mg/mL. A total of 12 formulations were designed, varying in buffer system, pH, protective component, and protein concentration. The samples were subjected to isothermal stability testing by incubating at 55°C for 21 hours, followed by evaluation of protein denaturation, aggregation, particle size, particle size distribution, and turbidity to improve the protein's long-term stability.

**Table 12 Formulation information**

| Formulation No. | Buffer System | pH | Protein Concentration (g/L) | Polysorbate 80 (ppm) | Trehalose (%) | Arginine Hydrochloride (mM) |
|---|---|---|---|---|---|---|
| A | 20 mM Histidine/Histidine Hydrochloride | 5.3 | 50±5 | 200 | 8 | 100 |
| 1-1 | | 5.0 | | | | |
| 1-2 | | 5.6 | | | | |
| 1-3 | | 6.0 | | | | |
| 1-4 | 10 mM Histidine/Histidine Hydrochloride | 5.3 | | | | |
| 1-5 | 20mM acetic acid/ sodium acetate | 5.0 | | | | |

The samples under six formulation conditions listed in Table 12 were incubated at 55°C for a certain period, and their turbidity (OD350) was measured at regular intervals using the turbidimetric method. The data of protein denaturation, aggregation, and particle size changes output by UNcle were analyzed to screen and rank the formulations, thereby determining the optimal formulation.

By comparing the denaturation, aggregation, particle size, and particle size distribution curves of the formulations with different buffer systems and pH levels, it was found that the protein exhibited better thermal stability in the histidine buffer system compared to the acetate buffer system. Additionally, the higher the pH and the lower the concentration of the buffer system, the less likely the protein was to undergo denaturation and aggregation, and the smaller the changes in particle size.

By comparing the turbidity changes of the formulations with different buffer systems and pH levels, it was found that the protein in the histidine buffer system showed smaller turbidity changes compared to the acetate buffer system. Moreover, the higher the pH and the lower the concentration of the buffer system, the less likely the protein's turbidity was to change.

Based on a comprehensive analysis of the UNcle and turbidity test results, 10 mM histidine and pH 6.0 were selected as the buffer system and pH for the formulation.

### 2.2 Screening Study of Protective Component in the Formulation

**Table 13 Formulation Information**

| Formulation No. | Buffer System | pH | Protein Concentration (g/L) | Polysorbate 80 (ppm) | Trehalose (%) | Arginine Hydrochloride (mM) |
|---|---|---|---|---|---|---|
| A | 20 mM Histidine/ Histidine Hydrochloride | 5.3 | 50±5 | 200 | 8 | 100 |
| 1-6 | | | | | 10 | 100 |
| 1-7 | | | | | 12 | 100 |
| 1-8 | | | | | 8 | 75 |
| 1-9 | | | | | 8 | 125 |

The samples under the five formulation conditions listed in Table 13 were incubated at 55°C for a certain period, and their turbidity (OD350) was measured at regular intervals using the turbidimetric method. The data of protein denaturation, aggregation, and particle size changes output by UNcle were analyzed to screen and rank the formulations, thereby determining the optimal formulation.

By comparing the denaturation, aggregation, particle size, and particle size distribution curves of the formulations with different concentrations of protective components, it was found that within the range of 8% to 12% trehalose, the higher the trehalose content, the less likely the protein was to undergo denaturation and aggregation, and the smaller the changes in particle size. Additionally, when the formulation contained 100 mM arginine hydrochloride, the protein exhibited slower denaturation and aggregation compared to formulations with 75 mM and 125 mM, indicating better thermal stability.

By comparing the turbidity changes of the formulations with different concentrations of protective components, it was found that within the range of 75 mM to 125 mM arginine hydrochloride, the higher the arginine hydrochloride concentrations, the smaller the turbidity changes of the protein. Furthermore, compared to trehalose concentrations of 8% and 12%, the protein exhibited smaller turbidity changes when the trehalose concentration was 10%.

Based on a comprehensive analysis of the UNcle and turbidity test results, and considering the appropriate osmolality of the formulation, 10% trehalose and 100 mM arginine hydrochloride were selected as the protective component for the formulation.

### 2.3 Screening of Surfactant Concentration

**Table 14 Formulation information**

| Formulation No. | Buffer System | pH | Protein Concentration (g/L) | Polysorbate 80 (ppm) | Trehalose (%) | Arginine Hydrochloride (mM) |
|---|---|---|---|---|---|---|
| A | 20 mM Histidine/ Histidine Hydrochloride | 5.3 | 50±5 | 200 | 8 | 100 |
| 1-10 | | | | 300 | | |
| 1-11 | | | | 500 | | |

The UNcle with isothermal stability function was used to incubate the samples under the above three formulation conditions at 55°C for a certain period. By comparing the rates of denaturation, aggregation, and particle size increase among the formulations, the optimal concentration of surfactant was screened.

By comparing the denaturation, aggregation, particle size, and particle size distribution curves of the formulations with different surfactant concentrations, it was found that compared to polysorbate 80 concentrations of 300 ppm and 500 ppm, when the concentration of polysorbate 80 was 200 ppm, the protein exhibited the slowest rates of denaturation and aggregation, indicating better thermal stability. Therefore, 200 ppm polysorbate 80 was selected as the surfactant for the formulation.

In summary, through a series of screening and optimization steps using high-throughput methods for the buffer system, pH, protective component, and surfactant in the formulation, the final formulation was determined to include the following: 10 mM histidine/histidine hydrochloride buffer system, pH 6.0, 10% trehalose, 100 mM arginine hydrochloride, 200 ppm polysorbate 80, with a protein concentration of 50 g/L.

### Example 6 Further Optimization Experiments for the Formulation

The formulation determination experiments in this example were conducted based on the preferred pH 6.0 from the high-throughput screening experiments (see Example 5), while also setting a lower limit of pH 5.3 and an upper limit of pH 6.3. This was to verify the long-term stability of the formulation within this pH range (i.e., 5.3-6.3) and to provide a controllable pH range for subsequent formulation production.

**Table 15 Formulation information**

| Formulation No. | Buffer System | pH | Protein Concentration (g/L) | Trehalose (%) | Arginine Hydrochloride (mM) | Polysorbate 80 (ppm) |
|---|---|---|---|---|---|---|
| 2-1 | 10 mM Histidine/ Histidine Hydrochloride | 5.3 | 50±5 | 10 | 100 | 200 |
| 2-2 | | 6.0 | | | | |
| 2-3 | | 6.3 | | | | |

The samples from Example 2 were ultrafiltered and exchanged into 10 mM histidine/histidine hydrochloride buffer systems with pH 5.3, pH 6.0, and pH 6.3, respectively, and then concentrated to a final protein concentration of no less than 50 mg/mL. Subsequently, protective components were added to prepare three different formulations as shown in Table 15. The samples under these three formulation conditions were sterilized by filtration through a 0.22 µm membrane and then aliquoted into vials for stability studies.

**Table 16 Stability Study**

| Testing Condition | 0-point | 2-week | 4-week | | 6-week |
|---|---|---|---|---|---|
| 2~8°C | + | - | + | | + |
| 25±2°C/60%±5%RH | | - | + | | + |
| 40±2°C/75%±5%RH | | + | + | | + |
| Freeze-thaw (-80°C freeze for 24h then thaw in 30°C water bath, counted as one cycle) | | 3 cycles | | 6 cycles | |
| | | + | | + | |

Samples from the three formulations in Table 15 were collected at the time points indicated in Table 16 and evaluated for appearance, SEC-HPLC, charge distribution (iCIEF), rCE-SDS purity, and nrCE-SDS purity. The results are shown in Tables 17~21.

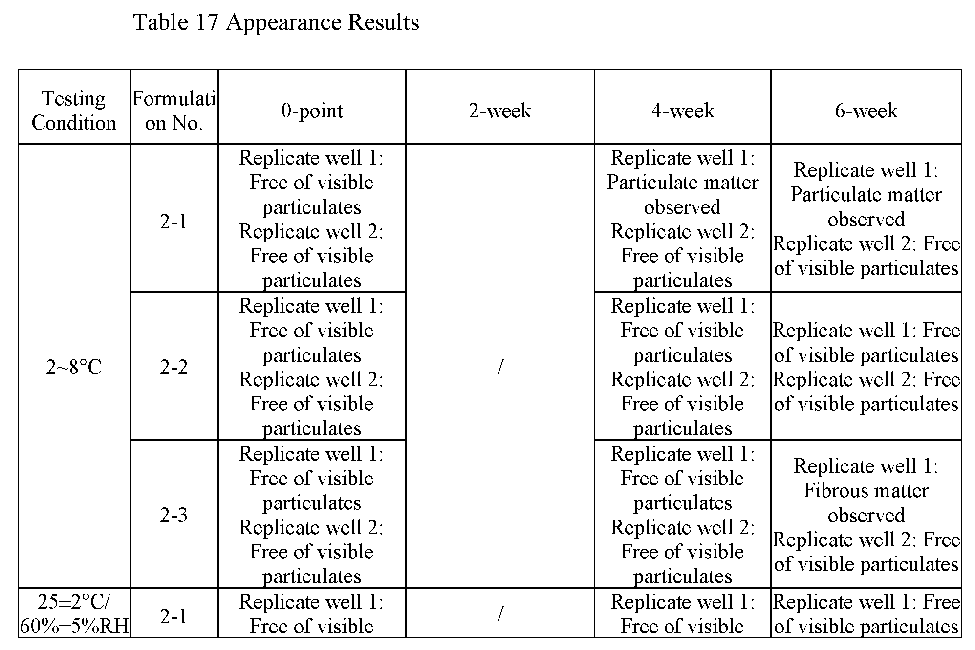

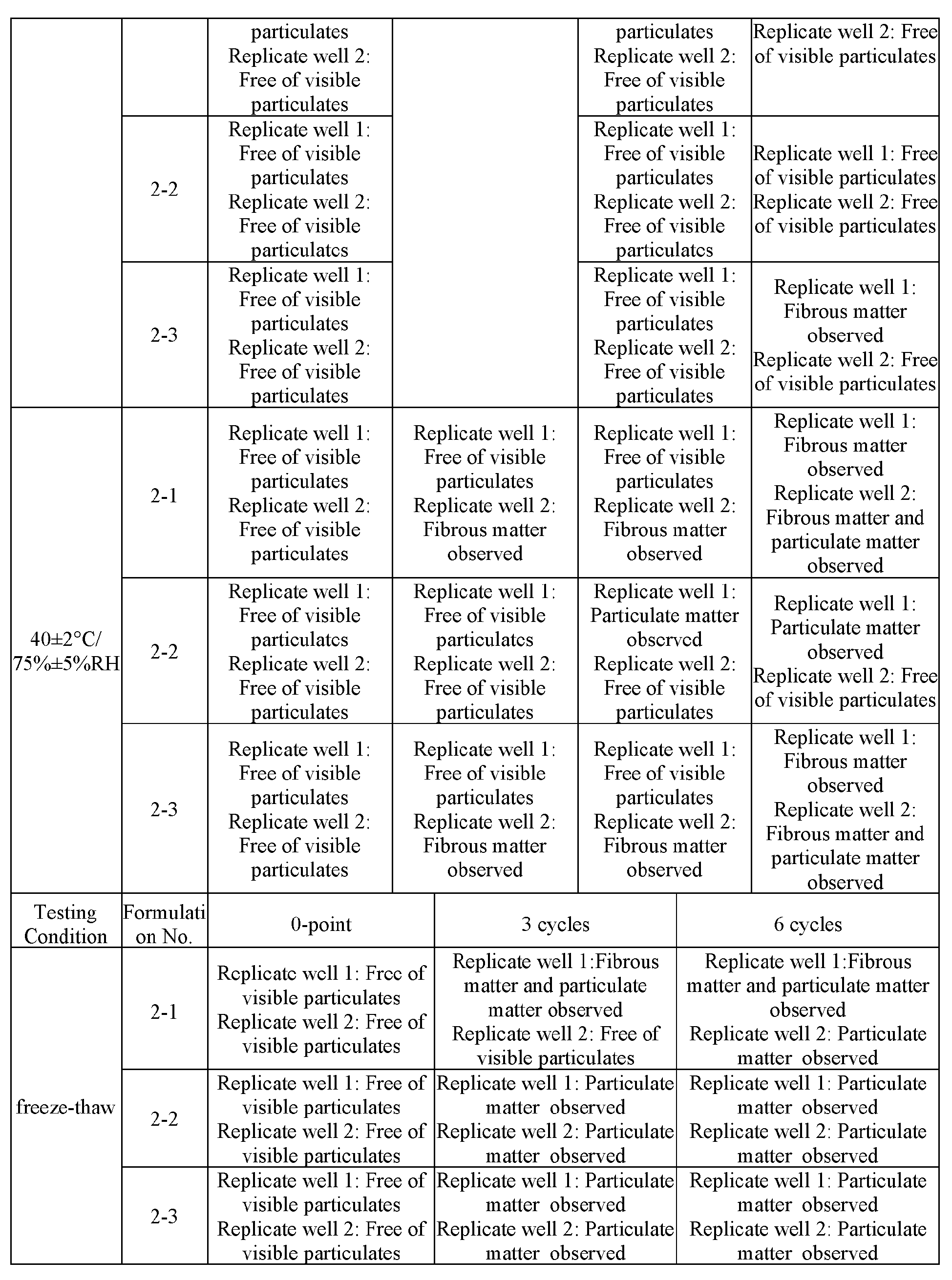

For formulations 2-1 and 2-3 stored at 2-8°C, visible particulates were observed in one replicate well after 6 weeks, while formulation 2-2 showed no visible particulates formation. At 25°C, formulation 2-3 exhibited visible particulates in one replicate well after 6 weeks. At 40°C, all formulations showed particulate formation after 6 weeks, with formulation 2-2 performing slightly better in terms of appearance. Under freeze-thaw conditions, all formulations developed visible particulates after just three cycles, indicating high sensitivity of the protein to freeze-thaw.

**Table 18 SEC-HPLC Results**

| 2~8°C | | | | | | | | | |
|---|---|---|---|---|---|---|---|---|---|
| Formulation No. | 0-point | 4-week | 6-week | 0-point | 4-week | 6-week | 0-point | 4-week | 6-week |
| | aggregate(%) | | | monomer(%) | | | fragment(%) | | |
| 2-1 | 0.6 | 0.7 | 0.7 | 99.4 | 99.1 | 99.2 | 0 | 0.2 | 0.1 |
| 2-2 | 0.4 | 0.4 | 0.4 | 99.6 | 99.4 | 99.4 | 0 | 0.2 | 0.1 |
| 2-3 | 0.5 | 0.5 | 0.5 | 99.5 | 99.3 | 99.4 | 0 | 0.3 | 0.1 |

| 25±2°C/60%±5%RH | | | | | | | | | |
|---|---|---|---|---|---|---|---|---|---|
| Formulation No. | 0-point | 4-week | 6-week | 0-point | 4-week | 6-week | 0-point | 4-week | 6-week |
| | aggregate(%) | | | monomer(%) | | | fragment(%) | | |
| 2-1 | 0.6 | 0.7 | 0.7 | 99.4 | 99.1 | 99.1 | 0 | 0.2 | 0.1 |
| 2-2 | 0.4 | 0.5 | 0.6 | 99.6 | 99.3 | 99.3 | 0 | 0.2 | 0.1 |
| 2-3 | 0.5 | 0.5 | 0.6 | 99.5 | 99.1 | 99.3 | 0 | 0.4 | 0.1 |

| 40±2°C/75%±5%RH | | | | | | | | | |
|---|---|---|---|---|---|---|---|---|---|
| Formulation No. | 0-point | 4-week | 6-week | 0-point | 4-week | 6-week | 0-point | 4-week | 6-week |
| | aggregate(%) | | | monomer(%) | | | fragment(%) | | |
| 2-1 | 0.6 | 2.7 | 4.1 | 99.4 | 96.7 | 94.4 | 0 | 0.7 | 1.5 |
| 2-2 | 0.4 | 2.1 | 2.9 | 99.6 | 97.6 | 95.5 | 0 | 0.4 | 1.6 |
| 2-3 | 0.5 | 1.6 | 2.7 | 99.5 | 98.0 | 96.1 | 0 | 0.4 | 1.2 |

| freeze-thaw | | | | | | | | | |
|---|---|---|---|---|---|---|---|---|---|
| Formulation No. | 0-point | 3 cycles | 6 cycles | 0-point | 3 cycles | 6 cycles | 0-point | 3 cycles | 6 cycles |
| | aggregate(%) | | | monomer(%) | | | fragment(%) | | |
| 2-1 | 0.6 | 0.6 | 0.6 | 99.4 | 99.4 | 99.4 | 0 | 0.1 | 0.0 |
| 2-2 | 0.4 | 0.4 | 0.4 | 99.6 | 99.5 | 99.5 | 0 | 0.0 | 0.1 |
| 2-3 | 0.5 | 0.4 | 0.4 | 99.5 | 99.5 | 99.5 | 0 | 0.0 | 0.1 |

Compared to the 0-point, the SEC-HPLC results of the three formulations at different pH levels showed no significant changes after six freeze-thaw cycles or after 6 weeks of storage at 2~8°C and 25°C, with no notable differences among the formulations.

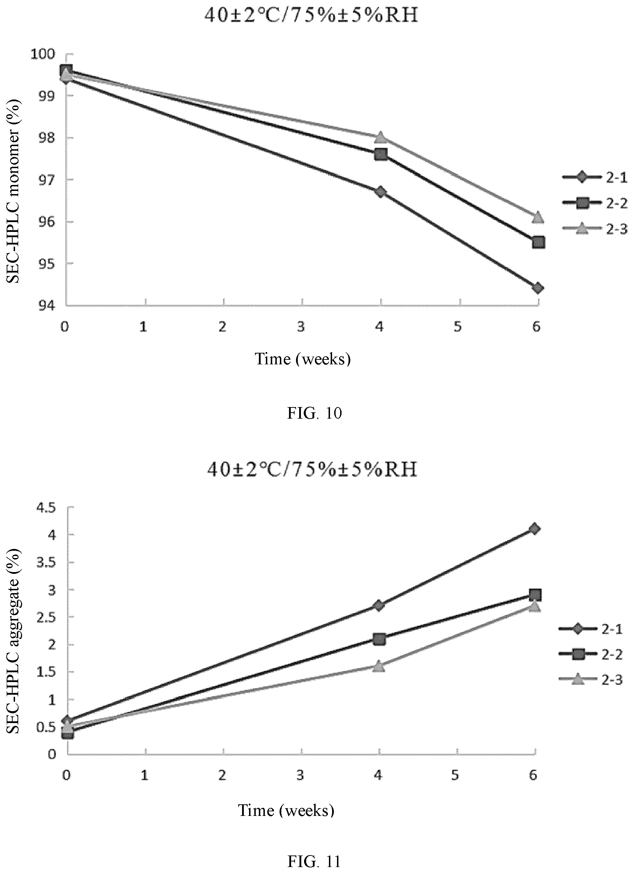

FIGS. 10 and 11 showed that after 6 weeks at 40°C, all formulations exhibited increased aggregates and decreased main peaks, with lower pH formulations demonstrating relatively faster aggregate formation.

**Table 19 iCIEF Results**

| 2~8°C | | | | | | | | | |
|---|---|---|---|---|---|---|---|---|---|
| Formulation No. | 0-point | 4-week | 6-week | 0-point | 4-week | 6-week | 0-point | 4-week | 6-week |
| | acidic peak (%) | | | main peak (%) | | | alkaline peak (%) | | |
| 2-1 | 26.6 | 33.7 | 34 | 54.0 | 50.3 | 49.7 | 19.4 | 15.9 | 16.3 |
| 2-2 | 27.0 | 34 | 33.8 | 52.7 | 50.4 | 50.8 | 20.3 | 15.6 | 15.4 |
| 2-3 | 26.9 | 34.4 | 34.7 | 54.4 | 50 | 49.3 | 18.7 | 15.6 | 16 |

| 25±2°C/60%±5%RH | | | | | | | | | |
|---|---|---|---|---|---|---|---|---|---|
| Formulation No. | 0-point | 4-week | 6-week | 0-point | 4-week | 6-week | 0-point | 4-week | 6-week |
| | acidic peak (%) | | | main peak (%) | | | alkaline peak (%) | | |
| 2-1 | 26.6 | 34.6 | 34.8 | 54.0 | 49.1 | 48.5 | 19.4 | 16.2 | 16.7 |
| 2-2 | 27.0 | 35 | 36.1 | 52.7 | 49.3 | 47.7 | 20.3 | 15.7 | 16.2 |
| 2-3 | 26.9 | 35.3 | 36.8 | 54.4 | 49 | 47.1 | 18.7 | 15.7 | 16.1 |

| 40±2°C/75%±5%RH | | | | | | | | | |
|---|---|---|---|---|---|---|---|---|---|
| Formulation No. | 0-point | 4-week | 6-week | 0-point | 4-week | 6-week | 0-point | 4-week | 6-week |
| | acidic peak (%) | | | main peak (%) | | | alkaline peak (%) | | |
| 2-1 | 26.6 | 43.9 | 51.2 | 54.0 | 38 | 30 | 19.4 | 18.1 | 18.7 |
| 2-2 | 27.0 | 48.7 | 54.1 | 52.7 | 34.9 | 30 | 20.3 | 16.4 | 15.9 |
| 2-3 | 26.9 | 49.5 | 55.8 | 54.4 | 35.1 | 29.4 | 18.7 | 15.5 | 14.8 |

| freeze-thaw | | | | | | | | | |
|---|---|---|---|---|---|---|---|---|---|
| Formulation No. | 0-point | 3 cycles | 6 cycles | 0-point | 3 cycles | 6 cycles | 0-point | 3 cycles | 6 cycles |
| | acidic peak (%) | | | main peak (%) | | | alkaline peak (%) | | |
| 2-1 | 26.6 | 26.7 | 30.9 | 54.0 | 53.6 | 49.4 | 19.4 | 19.6 | 19.7 |
| 2-2 | 27.0 | 26.6 | 30.1 | 52.7 | 55.2 | 51.2 | 20.3 | 18.2 | 18.7 |
| 2-3 | 26.9 | 26.5 | 30.4 | 54.4 | 55.1 | 50.6 | 18.7 | 18.4 | 18.9 |

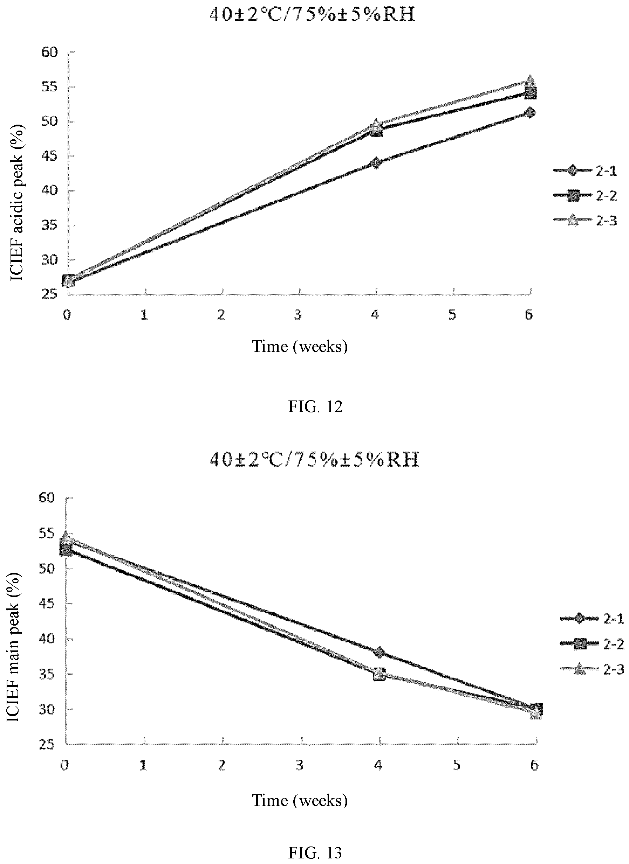

Compared to 0-point, after six freeze-thaw cycles or after 6 weeks of storage at 2-8°C and 25°C, the iCIEF acidic peaks of the three formulations at different pH levels showed an increasing trend, while the main peaks decreased. FIGS. 12 and 13 indicated that after 6 weeks at 40°C, the iCIEF acidic peaks of all formulations increased significantly, and the main peaks decreased notably, though no significant differences were observed among the formulations.

**Table 20 nrCE-SDS Purity Results**

| Formulati on No. | 0-point | 2~8°C | | 25+2°C/ 60%±5%RH | | 40±2°C/ 75%±5%RH | | freeze-thaw | |
|---|---|---|---|---|---|---|---|---|---|
| | | 4-week | 6-week | 4-week | 6-week | 4-week | 6-week | 3 cycles | 6 cycles |
| 2-1 | 97.4 | 97.6 | 97.6 | 97.3 | 97.7 | 94.9 | 92.5 | 97.6 | 97.1 |
| 2-2 | 97.5 | 97.8 | 97.6 | 97.3 | 97.3 | 94.9 | 93.5 | 97.4 | 97.1 |
| 2-3 | 97.5 | 97.6 | 97.6 | 97.3 | 97.2 | 94.9 | 93.1 | 97.3 | 97.2 |

Compared to 0-point, the nrCE-SDS results of the three formulations at different pH levels showed no significant changes after six freeze-thaw cycles or after 6 weeks of storage at 2~8°C and 25°C. After 6 weeks at 40°C, the nrCE-SDS main peaks of all formulations exhibited a slight downward trend, with no notable differences among the formulations.

**Table 21 rCE-SDS Purity Results**

| Formula tion No. | 0-point | 2~8°C | | 25±2°C/60%±5% RH | | 40±2°C/75%±5% RH | | freeze-thaw | |
|---|---|---|---|---|---|---|---|---|---|
| | | 4-week | 6-week | 4-week | 6-week | 4-week | 6-week | 3 cycles | 6 cycles |
| 2-1 | 96.9 | 94.6 | 96.1 | 96.4 | 96.1 | 95.3 | 93.9 | 96.4 | 96.0 |
| 2-2 | 96.7 | 96.8 | 96.0 | 96.2 | 96.0 | 95.0 | 94.5 | 96.2 | 96.3 |
| 2-3 | 96.7 | 96.3 | 96.2 | 96.2 | 96.0 | 95.3 | 94.7 | 96.2 | 96.3 |

The rCE-SDS results of the three formulations at different pH levels showed no significant changes after six freeze-thaw cycles or after 6 weeks of storage at 2~8°C and 25°C compared to 0-point. After 6 weeks at 40°C, the rCE-SDS main peaks of all formulations exhibited a slight downward trend, with no notable differences among the formulations.

Based on the comprehensive analysis of the physicochemical and appearance results, the protein demonstrated better appearance and more stable physicochemical properties at pH 6.0. Therefore, the final formulation pH range was determined to be 5.7~6.3, with a midpoint of pH 6.0. Additionally, since all three formulations at different pH levels were prone to forming visible particulates under freeze-thaw conditions, the protein concentration was reduced from 50 mg/mL to 30 mg/mL to improve stability .

Thus, the final SR604 protein formulation was determined to include the following: 10 mM histidine/histidine hydrochloride buffer system, pH 6.0 ± 0.3, 10% trehalose, 100 mM arginine hydrochloride, 200 ppm polysorbate 80, with a protein concentration of 30 mg/mL.

The above examples and embodiments described are for illustrative purposes only and shall not be construed as limiting the scope of protection of the present disclosure. It will be apparent to those skilled in the art that, without departing from the scope and spirit of the present disclosure, various modifications, improvements, or additions may be made based on the technical teachings herein, and such modifications and additions should be regarded as falling within the scope of protection of the present disclosure. Any slight substitutions, alterations, or equivalent variations that utilize the disclosed techniques to achieve substantially the same results are likewise considered equivalent embodiments of the present disclosure, and all such equivalents remain within the technical scope of the claims.

## Claims

1. A formulation of an anti-activated protein C (aPC) monoclonal antibody, wherein a buffer system of the formulation is histidine/histidine hydrochloride or acetic acid/sodium acetate;
wherein a protective component of the formulation comprises at least two of the following:
a) a sugar or sugar alcohol, wherein the sugar is trehalose or sucrose, and the sugar alcohol is sorbitol or mannitol;
b) an amino acid, wherein the amino acid is arginine hydrochloride or aspartic acid; and
c) sodium chloride;
wherein a pH of the formulation ranges from 5.0 to 6.3.

2. The formulation of claim 1, wherein the buffer system is 10 mM histidine/histidine hydrochloride, 20 mM histidine/histidine hydrochloride, or 20 mM acetic acid /sodium acetate;
preferably, the buffer system is 10 mM histidine/histidine hydrochloride or 20 mM histidine/histidine hydrochloride;
more preferably, the buffer system is 10 mM histidine/histidine hydrochloride;
or the content of trehalose ranges from 5 to 15% wt;
or the content of sucrose is 8% wt;
or the content of sorbitol is 6% wt;
or the content of mannitol is 6% wt;
or the content of arginine hydrochloride ranges from 50 to 150 mmol/L;
or the content of aspartic acid is 10 mmol/L;
or the content of sodium chloride ranges from 50 to 150 mmol/L.

3. The formulation of claim 1 or 2, wherein the protective component in the formulation is selected from the group consisting of: 8% trehalose + 100 mmol/L arginine hydrochloride, 6% sorbitol + 100 mmol/L arginine hydrochloride, 6% mannitol + 100 mmol/L arginine hydrochloride, 100 mmol/L arginine hydrochloride + 50 mmol/L sodium chloride, 100 mmol/L arginine hydrochloride + 10 mmol/L aspartic acid, 8% trehalose + 100 mmol/L sodium chloride, 8% trehalose + 100 mmol/L arginine hydrochloride, 8% trehalose + 100 mmol/L arginine hydrochloride + 50 mmol/L sodium chloride, 8% trehalose + 100 mmol/L arginine hydrochloride + 100 mmol/L sodium chloride, and 8% trehalose + 100 mmol/L arginine hydrochloride + 150 mmol/L sodium chloride;
preferably, the protective component in the formulation is 8% trehalose + 100 mmol/L arginine hydrochloride or 100 mmol/L arginine hydrochloride + 50 mmol/L sodium chloride.

4. The formulation of any one of claims 1 to 3, further comprising a surfactant, wherein the surfactant is polysorbate 80 and/or P188;
preferably, the surfactant is 200~500 ppm polysorbate 80, 200~500 ppm P188, or 200 ppm polysorbate 80 + 200 ppm P188;
more preferably, the surfactant is selected from 200 ppm polysorbate 80, 300 ppm polysorbate 80, 500 ppm polysorbate 80, 300 ppm P188, 500 ppm P188, and 200 ppm polysorbate 80 + 200 ppm P188;
more preferably, the surfactant is 200 ppm polysorbate 80 + 200 ppm P188.

5. The formulation of any one of claims 1 to 4, wherein the anti-aPC monoclonal antibody comprises a light chain having an amino acid sequence of SEQ ID NO: 1 and a heavy chain having an amino acid sequence of SEQ ID NO: 2, or the anti-aPC monoclonal antibody comprises a light chain having an amino acid sequence of SEQ ID NO: 3 and a heavy chain having an amino acid sequence of SEQ ID NO: 4;
preferably, the anti-aPC monoclonal antibody comprises a light chain having an amino acid sequence of SEQ ID NO: 1 and a heavy chain having an amino acid sequence of SEQ ID NO: 2.

6. The formulation of any one of claims 1 to 5, wherein the anti-aPC monoclonal antibody is of the IgG2 subtype or IgG4 subtype; preferably, the anti-aPC monoclonal antibody is of the IgG4 subtype.

7. The formulation of any one of claims 1 to 6, wherein the concentration of the anti-aPC monoclonal antibody in the formulation ranges from 30 to 70 mg/mL; preferably, the concentration of the anti-aPC monoclonal antibody is in a range of 27~33 mg/mL, 45~55 mg/mL, or 63~77 mg/mL.

8. The formulation of any one of claims 1 to 7, wherein the formulation comprises:
20 mM histidine/histidine hydrochloride, 8% trehalose, 100 mM arginine hydrochloride, 200 ppm polysorbate 80, and the anti-aPC monoclonal antibody having a concentration of 30 mg/mL, with a pH of 5.3; or
20 mM histidine/histidine hydrochloride, 6% sorbitol, 100 mM arginine hydrochloride, 200 ppm polysorbate 80, and the anti-aPC monoclonal antibody having a concentration of 30 mg/mL, with a pH of 5.3; or
20 mM histidine/histidine hydrochloride, 6% mannitol, 100 mM arginine hydrochloride, 200 ppm polysorbate 80, and the anti-aPC monoclonal antibody having a concentration of 30 mg/mL, with a pH of 5.3; or
20 mM histidine/histidine hydrochloride, 100 mM arginine hydrochloride, 50 mM sodium chloride, 200 ppm polysorbate 80, and the anti-aPC monoclonal antibody having a concentration of 30 mg/mL, with a pH of 5.3; or
20 mM histidine/histidine hydrochloride, 100 mM arginine hydrochloride, 10 mM aspartic acid, 200 ppm polysorbate 80, and the anti-aPC monoclonal antibody having a concentration of 30 mg/mL, with a pH of 5.3; or
20 mM histidine/histidine hydrochloride, 8% trehalose, 100 mM sodium chloride, 200 ppm polysorbate 80, and the anti-aPC monoclonal antibody having a concentration of 30 mg/mL, with a pH of 5.3; or
20 mM histidine/histidine hydrochloride, 8% trehalose, 100 mM arginine hydrochloride, 200 ppm polysorbate 80, and the anti-aPC monoclonal antibody having a concentration of 30 mg/mL, with a pH of 5.6.

9. The formulation of any one of claims 1 to 8, wherein the formulation comprises a 10 mM histidine/histidine hydrochloride buffer system, 10% trehalose, 100 mM arginine hydrochloride, 200 ppm polysorbate 80, and the anti-aPC monoclonal antibody having a concentration of 30 mg/mL, with a pH of 5.7~6.3.

10. Use of the anti-aPC monoclonal antibody formulation of any one of claims 1 to 9, in one or more of the following:
i) the preparation of a medicament for preventing and/or treating coagulation deficiency or defect disorders;
ii) the preparation of a promoter for enhancing the ability of aPC to cleave histones H3 and H4;
iii) the preparation of a medicament for inhibiting the anticoagulant activity of activated protein C in an individual;
iv) the preparation of a medicament for treating individuals requiring blood coagulation;
vi) the preparation of a medicament for regulating hemostasis in an individual;
vii) the immunological assay analysis for disease or non-disease diagnostic or therapeutic purposes, or the preparation of detection reagents or kits for aPC;
viii) combination with other reagents or drugs.

11. The use of claim 10, wherein the coagulation deficiency or defect disorders comprise disorders caused by coagulation factor deficiencies that lead to bleeding and coagulation abnormalities, as well as coagulopathies caused by trauma or surgery;
preferably, the coagulation deficiency or defect disorders comprise hemophilia, sepsis, and traumatic hemorrhage.
